# FASCICULE DE BREVET EUROPEEN

(11) **EP 0 533 573 B1**
(45) Date de publication et mention de la délivrance du brevet: **04.12.1996**
(21) Numéro de dépôt: 92402554.7
(22) Date de dépôt: 17.09.1992
(51) Int. Cl.: C07C 255/31, A61K 31/275, C07D 317/64, C07D 213/75, C07C 317/40

(54) **Nouveaux dérivés du 3-cyclo alkyl-propen-2-amide, leurs formes tautomères et leurs sels, procédé de préparation, application à titre de médicaments et compositions les renfermant**
Derivate des 3-Cyclo-alkylpropen-2-amide, deren Tautomere und Salze und ihre Verwendung als entzündungshemmende Wirkstoffe
Derivatives of 3-cyclo alkyl-propen-2-amide, their tautomeres and salts and their use as antiinflammatory agents

(30) Priorité: 17.09.1991 GB 9119874; 01.07.1992 GB 9213972
(43) Date de publication de la demande: 24.03.1993
(73) Titulaire: ROUSSEL UCLAF, 93230 Romainville (FR)
(72) Inventeur: Kuo, Elizabeth Anne, Swindon, Wilts SN3 5BZ (GB)
(74) Mandataire: Vieillefosse, Jean-Claude

(56) Documents cités:
- EP-A- 0 036 387
- EP-A- 0 257 882
- EP-A- 0 326 107
- DE-A- 2 555 789
- US-A- 4 435 407
- US-A- 5 034 410

## Description

La présente invention concerne de nouveaux dérivés du 3-cycloalkyl-propen-2-amide, leurs formes tautomères et leurs sels, ainsi que le procédé de préparation, l'application à titre de médicaments de ces nouveaux produits et les compositions les renfermant.

Dans US 5,034,410, EP-A 36387, DE-A 2555789 et EP-A 257882, des produits aromatiques sont décrits, qui ne contiennent pas de structure 3-cycloalkyl-propen-2-amide. EP-A 326107 enseigne des composés ayant une activité anti-inflammatoire.

L'invention a pour objet de nouveaux dérivés du 3-cycloalkyl-propen-2-amide répondant à la formule générale (I) : 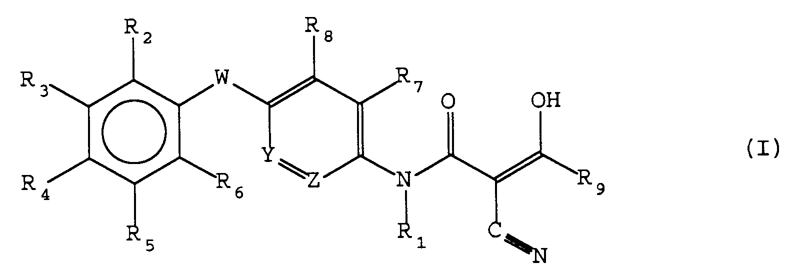 dans laquelle :
- R₁ représente un atome d'hydrogène ou un radical alkyle renfermant de 1 à 3 atomes de carbone,
- W représente un atome d'oxygène ou de soufre éventuellement oxydé sous forme de sulfoxyde ou de sulfone,
- Y et Z représentent un groupement -CH- ou l'un de Y et Z représente un groupement -CH- tandis que l'autre représente un atome d'azote,
- R₂, R₃, R₄, R₅ et R₆, identiques ou différents, représentent un atome d'hydrogène, un atome d'halogène, un radical alkyle renfermant de 1 à 4 atomes de carbone, un radical méthoxy, un radical méthylthio, un radical -WCF₃ dans lequel W a la signification indiquée ci-dessus, un groupement -CF₃ , un groupement NO₂, un groupement nitrile, un groupement -W(CH₂)ₙ-CF₃, -W(CF₂)ₙ-CF₃ et (CF₂)ₙ-CF₃ dans lequel W a la signification déja indiquée et n représente un nombre entier égal à 1, 2 ou 3, un groupement -(CH₂)ₙ-CX₃ dans lequel n a la signification déja indiquée et X représente un atome d'halogène, ou R₃ et R₄ forment ensemble un groupement -O-CH₂-O- et R₂, R₅, et R₆ ont la signification indiquée ci-dessus,
- R₇ et R₈, identiques ou différents représentent un atome d'hydrogène ou un radical alkyle renfermant de 1 à 6 atomes de carbone,
- R₉ représente un radical cycloalkyle renfermant de 3 à 6 atomes de carbone,
leurs formes tautomères, ainsi que leurs sels d'addition avec les bases minérales ou organiques.

Dans la formule générale (I) et dans ce qui suit :
- par radical alkyle renfermant de 1 à 4 atomes de carbone, on entend un radical méthyle, éthyle, propyle, isopropyle, n-butyle, isobutyle ou tert-butyle ;
- par radical alkyle renfermant de 1 à 6 atomes de carbone, on entend un radical méthyle, éthyle, propyle ou isopropyle, butyle linéaire ou ramifié, pentyle linéaire ou ramifié, hexyle linéaire ou ramifié ;
- par radical cycloalkyle renfermant de 3 à 6 atomes de carbone, on entend un radical cyclopropyle, cyclobutyle, cyclopentyle ou cyclohexyle ;
- par atome d'halogène, on entend un atome de fluor, de chlore, de brome ou d'iode, mais de préférence un atome de fluor, de chlore ou d'iode.

Les sels d'addition avec les bases minérales ou organiques, peuvent être, par exemple, les sels formés avec l'hydroxyde de sodium, le carbonate de potassium, l'éthanolamine ou la triéthylamine.

Parmi les produits, objet de l'invention, on peut citer notamment les dérivés répondant à la formule (I) ci-dessus, caractérisés en ce que dans ladite formule (I),
- R₁ représente un atome d'hydrogène ou un radical méthyle,
- W représente un atome d'oxygène,
- R₂, R₃, R₄, R₅ et R₆, identiques ou différents, représentent un atome d'hydrogène, un atome de fluor, de chlore, de brome ou d'iode, un radical méthyle, un radical méthoxy, un radical -OCF₃ , un radical -CF₃, un groupement NO₂, un groupement nitrile, un groupement -W(CH₂)ₙ-CF₃ dans lequel W et n ont la signification déja indiquée, ou, R₃ et R₄ forment ensemble un groupement -O-CH₂-O-, et R₂, R₅, et R₆ représentent un atome d'hydrogène,
- R₇ et R₈ représentent un atome d'hydrogène,
- R₉ représente un radical cyclopropyle,
- Y et Z ont la signification déja indiquée,
leurs formes tautomères, ainsi que leurs sels d'addition avec les bases minérales ou organiques.

Parmi ces derniers, on peut citer les dérivés répondant à la formule (I) ci-dessus, caractérisés en ce que dans ladite formule (I), R₁ représente un atome d'hydrogène, W représente un atome d'oxygène, R₂, R₃, R₄, R₅ et R₆, identiques ou différents, représentent un atome d'hydrogène, un atome de fluor, de chlore, d'iode ou un groupement NO₂, ou, R₃ et R₄ forment ensemble un groupement -O-CH₂-O-, et R₂, R₅ et R₆ représentent un atome d'hydrogène, R₇ et R₈ représentent un atome d'hydrogène et R₉ représente un radical cyclopropyle et Y et Z ont la signification déja indiquée, leurs formes tautomères, ainsi que leurs sels d'addition avec les bases minérales ou organiques.

Parmi ces derniers on retient tout particulièrement les dérivés de formule (I) dont les noms suivent :
- le N-[4-(4′-chlorophénoxy)phenyl]-2-cyano-3-cyclopropyl-3-hydroxy prop-2-enamide, ainsi que ses sels d'addition avec les bases minérales ou organiques,
- le 2-cyano-3-cyclopropyl-3-hydroxy-N-[4-(4′-nitrophénoxy)phenyl] prop-2-enamide, ainsi que ses sels d'addition avec les bases minérales ou organiques,
- le 2-cyano-3-cyclopropyl-3-hydroxy-N-[4-(3′,4′-methylene-dioxyphénoxy)phenyl] prop-2-enamide, ainsi que ses sels d'addition avec les bases minérales ou organiques,
- le 2-cyano-3-cyclopropyl-N-[4-(4′-fluorophénoxy)phenyl] 3-hydroxy prop-2-enamide, ainsi que ses sels d'addition avec les bases minérales ou organiques,
- le 2-cyano-3-cyclopropyl-3-hydroxy-N-[4-(4′-iodophénoxy)phenyl] prop-2-enamide, ainsi que ses sels d'addition avec les bases minérales ou organiques.

L'invention a également pour objet un procédé de préparation des nouveaux dérivés du 3-cyclopropyl-propen-2-amide tels que définis par la formule (I) ci-dessus, ainsi que de leurs sels d'addition avec les bases minérales ou organiques, caractérisé en ce que l'on fait réagir un produit de formule (II) : 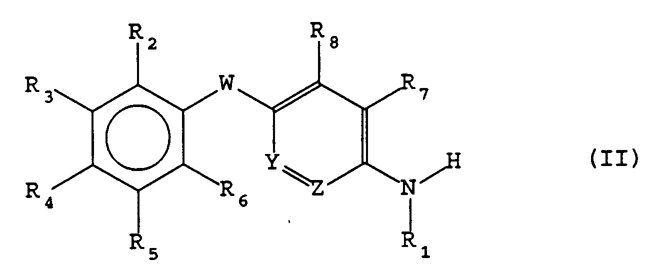
dans laquelle R_{**1**}, R_{**2**}, R_{**3**}, R_{**4**}, R_{**5**}, R_{**6**}, R_{**7**}, R_{**8**}, W, Y et Z ont la signification déja indiquée, avec un produit de formule (III):

HO-CO-CH_{**2**}-CN (III)

pour obtenir un produit de formule (IV) : 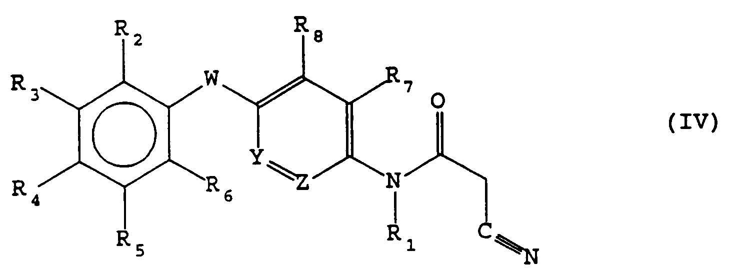
dans laquelle R_{**1**}, R_{**2**}, R_{**3**}, R_{**4**}, R_{**5**}, R_{**6**}, R_{**7**}, R_{**8**}, W, Y et Z ont la signification déja indiquée, puis, fait réagir ce dernier, successivement avec de l'hydrure de sodium, puis avec un produit de formule (V):

Hal-CO-R_{**9**} (V)

dans laquelle Hal représente un atome d'halogène et R₉ a la signification déjà indiquée, pour obtenir le produit de formule (I) recherché, que l'on peut salifier si désiré.

Dans des conditions préférentielles de mise en oeuvre de l'invention, le procédé de préparation ci-dessus décrit est caractérisé en ce que :
- la réaction du produit de formule (II) avec le produit de formule (III) est effectuée en présence de pentachlorure de phosphore au sein d'un solvant organique anhydre, tel que le chlorure de méthylène ou le tétrahydrofurane,
- la réaction du produit de formule (IV) avec l'hydrure de sodium est éffectuée au sein d'un solvant organique anhydre tel que le tétrahydrofurane, le cas échéant en présence d'un catalyseur tel qu'un imidazole.

Les produits de formule (I) présentent un caractère acide. On peut avantageusement préparer les sels d'addition des produits de formule (I) en faisant réagir, en proportions sensiblement stoéchiométriques, un base minérale ou organique avec lesdits produits de formule (I). Les sels peuvent être préparés sans isoler les bases correspondantes.

Les produits, objet de la présente invention, possèdent de très intéressantes propriétés pharmacologiques. On note en particulier une remarquable activité anti-inflammatoire. Ils inhibent d'une part les phénomènes inflammatoires provoqués par des agents irritants, et d'autre part les réactions d'hypersensibilité retardée, en empéchant l'activation des cellules immunitaires par un antigène spécifique.

Ces propriétés sont illustrées plus loin dans la partie expérimentale.

Ces propriétés justifient l'utilisation des nouveaux dérivés du 3-cycloalkyl-propen-2-amide, ainsi que de leurs sels d'addition avec les bases pharmaceutiquement acceptables, à titre de médicaments.

La présente invention a aussi également pour objet l'application à titre de médicaments des nouveaux dérivés du 3-cycloalkyl-propen-2-amide, tels que définis par la formule générale (I), ainsi que de leurs sels d'addition avec les bases minérales ou organiques pharmaceutiquement acceptables.

Parmi les médicaments, objet de l'invention, on retient notamment les médicaments, caractérisés en ce qu'ils sont constitués par les nouveaux dérivés du 3-cycloalkyl-propen-2-amide répondant à la formule (I) ci-dessus dans laquelle R₁ représente un atome d'hydrogène ou un radical méthyle,
- W représente un atome d'oxygène,
- R₂, R₃, R₄, R₅ et R₆, identiques ou différents, représentent un atome d'hydrogène, un atome de fluor, de chlore, de brome ou d'iode, un radical méthyle, un radical méthoxy, un radical -OCF₃ , un radical -CF₃ , un groupement NO₂, un groupement nitrile, un groupement -W(CH₂)ₙ-CF₃ dans lequel W et n ont la signification déja indiquée,
ou, R₃ et R₄ forment ensemble un groupement -O-CH₂-O-, et R₂, R₅, et R₆ représentent un atome d'hydrogène,
R₇ et R₈ représentent un atome d'hydrogène,
R₉ représente un radical cyclopropyle,
Y et Z ont la signification déja indiquée,
leurs formes tautomères, ainsi que leurs sels d'addition avec les bases minérales ou organiques pharmaceutiquement acceptables.

Parmi les médicaments, objet de l'invention, on retient plus particulièrement ceux répondant à la formule (I) ci-dessus dans laquelle R₁ représente un atome d'hydrogène, W représente un atome d'oxygène, R₂, R₃, R₄, R₅ et R₆, identiques ou différents, représentent un atome d'hydrogène, un atome de fluor, de chlore, d'iode ou un groupement NO₂, ou, R₃ et R₄ forment ensemble un groupement -O-CH₂-O-, et R₂, R₅ et R₆ représentent un atome d'hydrogène, R₇ et R₈ représentent un atome d'hydrogène et R₉ représente un radical cyclopropyle et Y et Z ont la signification déja indiquée, ainsi que leurs sels d'addition avec les bases minérales ou organiques pharmaceutiquement acceptables.

Parmi les médicaments préférés de l'invention, on retient tout particulièrement :
- la N-[4-(4′-chlorophénoxy)phenyl]-2-cyano-3-cyclopropyl-3-hydroxy prop-2-enamide, ainsi que ses sels d'addition avec les bases minérales ou organiques pharmaceutiquement acceptables,
- la 2-cyano-3-cyclopropyl-3-hydroxy-N-[4-(4-nitrophénoxy)phenyl] prop-2-enamide, ainsi que ses sels d'addition avec les bases minérales ou organiques pharmaceutiquement acceptables,
- la 2-cyano-3-cyclopropyl-3-hydroxy-N-[4-(3′,4′-methylene-dioxyphénoxy)phenyl] prop-2-enamide, ainsi que ses sels d'addition avec les bases minérales ou organiques pharmaceutiquement acceptables,
- la 2-cyano-3-cyclopropyl-N-[4-(4′-fluorophénoxy)phenyl] 3-hydroxy prop-2-enamide, ainsi que ses sels d'addition avec les bases minérales ou organiques pharmaceutiquement acceptables,
- la 2-cyano-3-cyclopropyl-3-hydroxy-N-[4-(4′-iodophénoxy)phenyl] prop-2-enamide, ainsi que ses sels d'addition avec les bases minérales ou organiques pharmaceutiquement acceptables.

Ces médicaments trouvent, par exemple, leur emploi dans le traitement de l'arthrite rhumatoïde et des maladies inflammatoires chroniques d'origine immune ou non, des troubles et maux survenant lors des transplantations, greffes et autres maladies liées à l'immunologie.

La dose usuelle variable selon le produit utilisé, le sujet traité et l'affection en cause peut être par exemple, de 0,1 mg à 200 mg par jour, par voie orale.

L'invention a également pour objet les compositions pharmaceutiques qui renferment au moins un dérivé précité ou l'un de ses sels d'addition avec les bases pharmaceutiquement acceptables à titre de principe actif.

A titre de médicaments, les dérivés répondant à la formule (I) et leurs sels d'addition avec les bases pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés simples ou dragéifiés, les gélules, les capsules, les granulés, les suppositoires, les préparations injectables ; elles sont préparées selon les méthodes usuelles. Le ou les principes actifs peuvent être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a également pour objet, à titre de produits industriels nouveaux et notamment à titre de produits industriels nécessaires comme intermédiaires pour la préparation des produits de formule (I) :
- les produits de formule (IV): 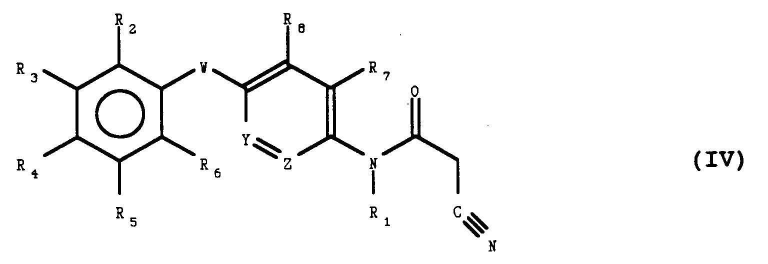 dans laquelle R₁, R₂, R₃, R₄, R₅, R₆, R₇, R₈, W, Y et Z ont la signification déjà indiquée et l'un de Y et Z représente un groupement -CH- tandis que l'autre représente un atome d'azote.

Les produits de formule (II), lorsqu'ils ne sont pas connus, peuvent être préparés par un procédé, caractérisé en ce que l'on réduit un produit de formule (VI) : 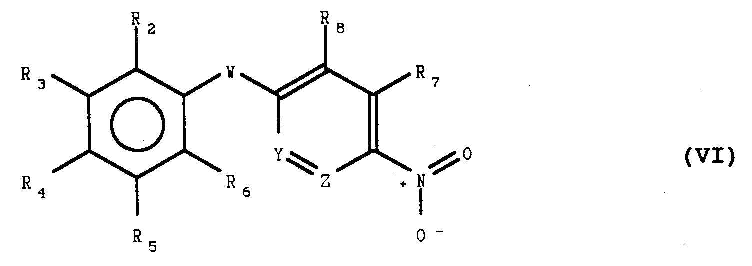 dans laquelle R₂, R₃, R₄, R₅, R₆, R₇, R₈, W, Y et Z ont la signification déja indiquée, pour obtenir le produit recherché de formule (II). La réduction peut être effectuée au moyen de l'hydrogène en présence d'un catalyseur ou au moyen d'acide chlorhydrique en présence de limaille de fer. Un exemple d'une telle préparation figure ci-après dans la partie expérimentale.

Les produits de formule (VI) peuvent eux-mêmes être préparés par réaction d'un produit de formule (VII) : 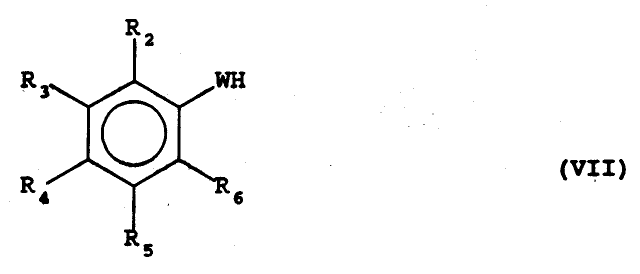
dans laquelle W, R₂, R₃, R₄, R₅, et R₆ ont la signification déjà indiquée, avec un produit de formule (VIII): 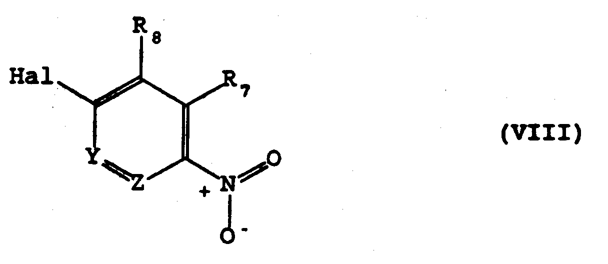
dans laquelle Hal, R₇, R₈, Y et Z ont la signification déja indiquée.

Les produits de formule (VI) dans laquelle W représente un groupement SO ou SO₂ peuvent être préparés par oxydation du produit de formule (VI) correspondant dans laquelle W représente un atome de soufre soit.

Il va être donné maintenant à titre non limitatif, des exemples de mise en oeuvre de l'invention.

### Exemple 1 : N-[4-(4′-chlorophénoxy) phényl] 2-cyano 3-cyclopropyl 3-hydroxy prop-2-énamide.

### Stade A : 4-(4′-chlorophénoxy) nitrobenzène.

On ajoute 10,88 g de carbonate de potassium et 9,64 g de 4-chlorophénol en suspension dans 100 ml de diméthylsulfoxyde à une solution comprenant 10,58 g de 1-fluoro 4-nitrobenzène dans 100 ml de diméthylsulfoxyde. On chauffe la suspension à 70°C pendant 4 heures, laisse revenir à température ambiante, ajoute 250 ml d'eau puis 250 ml d'acétate d'éthyle, on sépare la phase organique, la lave à l'eau puis à l'eau salée, sèche, filtre et évapore sous pression réduite pour obtenir 19,25 g de produit brut que l'on chromatographie sur silice en éluant au chlorure de méthylène. On obtient 18,34 g de produit attendu.

### Stade B : 4-(4′-chlorophénoxy) aniline.

16,0 g de produit obtenu ci-dessus et 161 mg d'oxyde de platine (Pt : 75%) en suspension dans 200 ml d'éthanol sont hydrogénés pendant 4 heures. On filtre sur célite et évapore le solvant. On obtient 13,98 g de produit attendu.

### Stade C : N-[4-(4′-chlorophénoxy) phényl] 2-cyano éthanamide.

On ajoute en 30 minutes 8,06 g d'acide cyano acétique à une suspension comprenant 19,73 g de pentachlorure de phosphore dans 150 cm³ de chlorure de méthylène sous agitation en maintenant à température ambiante. On chauffe au reflux 1 heure sous courant d'azote, ajoute en 30 minutes, 13,88 g de 4-(4′-chlorophénoxy) aniline dans 150 ml de chlorure de méthylène, agite la suspension pendant 1 heure et demie et laisse revenir à température ambiante, verse 250 ml d'eau et agite 1 heure. On élimine l'eau, agite 1 heure la suspension avec 250 ml d'une solution aqueuse saturée en bicarbonate de sodium. On ajoute de l'acétate d'éthyle jusqu'à dissolution du solide et sépare les phases. On lave la phase organique à l'eau salée, sèche, filtre et évapore le solvant. On obtient 17,49 g de produit attendu.

### Stade D : N-[4-(4′-chlorophénoxy) phényl] 2-cyano 3-cyclopropyl 3-hydroxy prop-2-énamide.

A 7 g de produit obtenu au stade C en suspension dans 80 ml de tétrahydrofuranne, on ajoute 2,20 g d'hydrure de sodium dans 5 ml de tétrahydrofuranne et agite 1 heure et demie à température ambiante. On ajoute en 20 minutes 3,32 g (2,88 ml) de chlorure de cyclopropanecarbonyle dans 10 ml de tétrahydrofuranne et agite le mélange 20 minutes à température ambiante. On ajoute 150 ml d'acide chlorhydrique 2N et 350 ml d'eau glacée. On agite 10 minutes la suspension, filtre et sèche sous pression réduite. On reprend le résidu dans 150 ml d'éther, agite 30 minutes, filtre, lave à l'éther de pétrole, sèche sous pression réduite et obtient 5,51 g de produit attendu.
F = 160,5°-162,5°C.

| Analyse : C₁₉H₁₅ClN₂O₃ = 354,80 | | | | | |
|---|---|---|---|---|---|
| Calculé | C% 64,32 | H% 4,26 | Cl% 9,99 | N% 7,90 | O% 13,53 |
| Trouvé | 63,95 | 4,31 | 10,26 | 7,91 | 13,57 |

Spectre RMN CDCl₃ :
1,11-1,24 (2H,m) ; 1,27-1,37 (2H,m) ; 2,09-2,19 (1H,m) ; 6,94 (2H, d+v, J=8,8) ; 7,00 (2H, d+v, J=8,8) ; 7,30 (2H, d+v, J=8,8) ; 7,44 (2H, d+v, J=8,8) ; 7,51 (1H, br s) et 15,84 (1H,s).
Spectre IR :
3272 (m), 2217 (m), 1544 (s), 1501 (s), 1482 (s), 1324 (m), 1350 (m), 1286 (m), 1258 (m), 1234 (s), 1196 (m), 1086 (m), 900 (m), 878 (m) et 823 (m).

En opérant selon le mode opératoire indiqué ci-dessus, à partir des composés appropriés, on a préparé les produits des exemples suivants :

### Exemple 2 : 2-cyano 3-cyclopropyl 3-hydroxy (4-phénoxyphényl) propèn-2-amide.

F = 142,0°C.

| Analyse : C₁₉H₁₆N₂O₃ = 320,35 | | | | |
|---|---|---|---|---|
| Calculé | C% 71,24 | H% 5,03 | N% 8,74 | O% 14,98 |
| Trouvé | 71,11 | 5,13 | 8,73 | |

### Exemple 3 : 2-cyano 3-cyclopropyl 3-hydroxy N-[4-(4-nitrophénoxy) phényl] propèn-2-amide.

F = 197,0°-198,0°C.

| Analyse : C₁₉H₁₅N₃O₅ = 365,35 | | | | |
|---|---|---|---|---|
| Calculé | C% 62,46 | H% 4,14 | N% 11,50 | O% 21,90 |
| Trouvé | 62,32 | 4,23 | 11,44 | |

Spectre RMN (DMSO) :
10,56 (1H,s) ; 8,30 (2H,d) ; 7,68 (2H,d) ; 7,23 (2H,d) ; 7,16 (2H,d) ; 2,22 (1H,m) ; 1,17 (4H,m).
Spectre IR :
3380, 2220, 1590, 1550, 1505, 1425, 1350, 1260, 1235, 1195, 1170, 1115, 1015, 990.

### Exemple 4 : 2-cyano 3-cyclopropyl 3-hydroxy N-[4-(3′,4′-méthylènedioxyphénoxy) phényl] prop-2-énamide.

F = 150,5°-151,5°C.

| Analyse : C₂₀H₁₆N₂O₅ = 364,36 | | | | |
|---|---|---|---|---|
| Calculé | C% 65,93 | H% 4,43 | N% 7,69 | O% 21,96 |
| Trouvé | 65,74 | 4,46 | 7,67 | 22,13 |

Spectre RMN (CDCl₃) :
1,09-1,21 (2H,m) ; 1,25-1,36 (2H,m) ; 2,09-2,20 (1H,m) ; 5,98 (2H,s) ; 6,49 (1H,AB q, J=8,0-2,4) ; 6,57 (1H,d, J=2,4) ; 6,76 (1H,d, J=8,2) ; 6,96 (2H, d+v, J=9,0) ; 7,38 (2H, d+v, J=8,8); 7,47 (1H, Br s) et 15,89 (1H,s).
Spectre IR :
3250 (s), 2209 (s), 1574 (s), 1539 (s), 1500 (s), 1481 (s), 1240 (m), 1214 (s), 1170 (m), 1031 (m), 926 (m).

### Exemple 5 : 2-cyano 3-cyclopropyl N-[4-(4′-fluorophénoxy) phényl] 3-hydroxy prop-2-énamide.

F = 135,5°-136,5°C.

| Analyse : C₁₉H₁₅FN₂O₃ = 338,34 | | | | | |
|---|---|---|---|---|---|
| Calculé | C% 67,45 | H% 4,47 | F% 5,62 | N% 8,28 | O% 14,19 |
| Trouvé | 67,25 | 4,53 | 5,65 | 8,24 | 14,33 |

Spectre RMN (CDCl₃) :
1,10-1,21 (2H,m) ; 1,27-1,36 (2H,m) ; 2,07-2,20 (1H,m) ; 6,93-7,09 (6H,m) ; 7,41 (2H, d+v, J=8,8); 7,48 (1H, Br s) et 15,85 (1H,s).
Spectre IR :
3280 (m), 2220 (m), 1577 (s), 1541 (s), 1496 (s), 1422 (m), 1412 (m), 1352 (m), 1291 (m), 1257 (m), 1228 (m), 1211 (s), 1188 (m), 1160 (m), 992 (m), 899 (m), 8,79 (m), 8,49 (m), 824 (m), 814 (m), 764 (m).

### Exemple 6 : 2-cyano 3-cyclopropyl 3-hydroxy N-[4-(4′-iodophénoxy) phényl] prop-2-énamide.

On opère comme à l'exemple 1 stade C et D en utilisant la 4-(4′-iodophénoxy) aniline préparée comme indiqué ci-dessous et en incorporant le produit obtenu au stade B sous forme de solide dans la solution tétrahydrofuranne d'hydrure de sodium. Après purification du produit brut obtenu au stade D à l'aide de méthanol, on obtient le produit attendu.
F = 162,0°-164,0°C.

| Analyse : C₁₉H₁₅IN₂O₃ = 446,25 | | | | | |
|---|---|---|---|---|---|
| Calculé | C% 51,14 | H% 3,39 | I% 28,44 | N% 6,28 | O% 10,76 |
| Trouvé | 51,00 | 3,43 | 28,42 | 6,30 | 10,85 |

Spectre RMN (CDCl₃) :
1,10-1,37 (4H,m) ; 2,11-2,21 (1H,m) ; 6,77 (2H, d+v, J=9,0); 7,01 (2H, d+v J=8,8) ; 7,44 (2H, d+v,J=9,0) ; 7,51 (1H,s) ; 7,62 (2H, d+v, J=8,8) 15,83 (1H,s).
Spectre IR :
3265 (m), 2212 (m), 1576 (s), 1530 (s), 1500 (s), 1477 (s), 1421 (m), 1406 (m), 1351 (m), 1272 (m), 1251 (m), 1233 (s), 1195 (m), 1163 (m), 1005 (m), 900 (m), 874 (m), 815 (m).

### Préparation de la 4-(4′-iodophénoxy) aniline.

On ajoute lentement en 50 minutes, 6,04 ml d'acide chlorhydrique concentré en solution dans 25 ml d'éthanol et 25 ml d'eau, dans 23,34 g de 4-(4′-iodophénoxy) nitrobenzène et 11,46 g de limaille de fer en suspension dans 150 ml d'éthanol et 150 ml d'eau et chauffe 1 heure au reflux. On refroidit à température ambiante, évapore partiellement (≈ 200 ml), alcalinise jusqu'à pH 11 à l'aide d'une solution aqueuse d'hydroxyde de sodium puis ajoute 200 ml d'eau et 250 ml d'acétate d'éthyle. On extrait la phase aqueuse à l'acétate d'éthyle, réunit les phases organiques, les lave à l'eau puis à l'eau salée, sèche et évapore le solvant. On obtient 20,25 g de produit brut que l'on purifie par chromatographie sur silice (éluant : chlorure de méthylène comprenant 0 à 10% d'acétate d'éthyle). On recueille 13,40 g de produit attendu.

### Exemple 7 : N-[[2-(4′-chlorophénoxy) pyridin-5-yl] 2-cyano 3-cyclopropyl 3-hydroxy prop-2-énamide.

En utilisant au départ la 2-chloro 5-nitropyridine et le 4-chlorophénol et en opérant comme à l'exemple 1 avec les modifications aux stades C et D telles qu'elles sont indiquées à l'exemple 6, on a préparé le produit attendu.
F = 198,0°-199,0°C.

| Analyse : C₁₈H₁₄ClN₃O₃ = 355,78 | | | | | |
|---|---|---|---|---|---|
| Calculé | C% 60,77 | H% 3,97 | Cl% 9,96 | N% 11,81 | O% 13,49 |
| Trouvé | 60,42 | 4,00 | 9,97 | 11,80 | 13,81 |

Spectre RMN (CDCl₃) :
1,12-1,38 (4H,m) ; 2,07-2,22 (1H,m) ; 6,96 (1H,d, J=8,8) ; 7,08 (2H, d+v, J=8,8); 7,52 (1H, Br s) ; 7,92 (1H,dd, J=8,8-2,8) ; 8,21 (1H,d, J=2,8) 15,61 (1H,s).
Spectre IR :
3285 (m), 2218 (m), 1614 (m), 1572 (m), 1542 (s), 1487 (m), 1469 (s), 1343 (m), 1259 (s), 1228 (s), 1203 (m), 1083 (m), 991 (m), 983 (m).

En opérant selon le mode opératoire indiqué à l'exemple 1 ci-dessus, à partir des composés appropriés, on a préparé les produits des exemples suivants :

### Exemple 8 : 2-cyano 3-cyclopropyl 3-hydroxy N-[4-(4′-trifluorométhylphénoxy ) phényl] prop-2-énamide.

Rendt. 71%.
F = 150,5°-152,0°C.

| Analyse : C₂₀H₁₅F₃N₂O₃ = 388,35 | | | | | |
|---|---|---|---|---|---|
| Calculé | C% 61,86 | H% 3,89 | F% 14,68 | N% 7,21 | O% 12,36 |
| Trouvé | 61,82 | 3,96 | 14,55 | 7,19 | 12,48 |

Spectre RMN (CDCl₃) :
1,11-1,38 (4H,m) ; 2,09-2,24 (1H,m) ; 7,05 (2H,d, J=9) ; 7,06 (2H, d+v, J=9); 7,49 (2H, d+v, J=9) ; 7,53 (1H,s) ; 7,59 (2H,d, J=9) 15,7 (1H,s).
Spectre IR :
3280 (m), 2218 (s), 1609 (s), 1577 (s), 1551 (s), 1502 (s), 1421 (m), 1335 (s), 1313 (s), 1293 (m), 1258 (s), 1232 (s), 1198 (m), 1170 (m), 1114 (m), 1103 (s), 1068 (s), 1012 (m), 897 (m), 878 (m), 850 (m), 835 (s).

### Exemple 9 : 2-cyano 3-cyclopropyl N-[4-(3′,4′-diméthoxyphénoxy) phényl] 3-hydroxy prop-2-énamide.

Rendt. 79%.
F = 154,0°-156,0°C.

| Analyse : C₂₁H₂₀N₂O₅ = 380,40 | | | | |
|---|---|---|---|---|
| Calculé | C% 66,31 | H% 5,30 | N% 7,36 | O% 21,03 |
| Trouvé | 66,09 | 5,50 | 7,16 | 21,25 |

Spectre RMN (CDCl₃) :
1,15 (2H,m) ; 1,31 (2H,m) ; 2,15 (1H,m) ; 3,84 (3H,s); 3,89 (3H,s) ; 6,56 (1H,dd, J=2,8-8,6) ; 6,64 (1H,d, J=2,4) ; 6,83 (1H,d J=8,6) ; 6,96 (2H,d J=8,8) ; 7,39 (2H,d J=9,0) ; 7,54 (1H,s) ; 15,39 (1H,s).
Spectre IR :
3380 (m), 2212 (m), 1540 (s), 1500 (s), 1220 (s).

### Exemple 10 : 2-cyano 3-cyclopropyl N-[4-(4'-chloro 3'-méthylphénoxy) phényl] 3-hydroxy prop-2-énamide.

Rendt. 75%.
F = 154,0°-156,0°C.

| Analyse : C₂₀H₁₇ClN₂O₃ = 368,82 | | | | | |
|---|---|---|---|---|---|
| Calculé | C% 65,13 | H% 4,65 | Cl% 9,61 | N% 7,60 | O% 13,01 |
| Trouvé | 65,18 | 4,79 | 9,65 | 7,42 | 12,96 |

Spectre RMN (CDCl₃) :
1,10-1,36 (4H,m) ; 2,09-2,34 (1H,m) ; 2,34 (3H,s) ; 6,78 (1H,dd, J=8,6-2,8); 6,88 (1H,d, J=2,8) ; 6,99 (2H,d, J=8,8) ; 7,28 (1H,d, J=8,6) ; 7,42 (2H,d J=8,8) ; 7,51 (1H,s) ; 15,6 (1H,s).
Spectre IR :
3264 (m), 2200 (m), 1600 (m), 1570 (m), 1563 (m), 1535 (s), 1500 (s), 1468 (s), 1408 (m), 1340 (m), 1292 (m), 1265 (m), 1220 (m), 1198 (m), 885 (m), 843 (m), 798 (m).

### Exemple 11 : 2-cyano 3-cyclopropyl N-[4-(4'-chloro 2'-méthylphénoxy) phényl] 3-hydroxy prop-2-énamide.

Rendt. 60%.
F = 128,0°-129,0°C.
Spectre RMN (CDCl₃) :
1,10-1,36 (4H,m) ; 2,08-2,16 (1H,m) ; 2,21 (3H,s) ; 6,83 (1H,d, J=8,8); 6,89 (2H,d, J=9) ; 7,13 (1H,dd, J=8,6-2,8) ; 7,24 (1H,d, J=2,4) ; 7,39 (2H,d J=8,8) ; 7,48 (1H,s) ; 15,87 (1H,s).
Spectre IR : 3270 (m), 2180 (m), 1595 (m), 1570 (s), 1535 (s), 1490 (s), 1465 (s), 1400 (m), 1335 (m), 1215 (s), 1190 (s), 1165 (s), 880 (m), 855 (m), 815 (m), 800(m).

### Exemple 12 : 2-cyano 3-cyclopropyl N-[4-(4'-chlorophénoxy) 3-méthylphényl] 3-hydroxy prop-2-énamide.

Rendt. 66%.

F = 124,0°-125,0°C.

| Analyse : C₂₀H₁₇ClN₂O₃ = 368,82 | | | | | |
|---|---|---|---|---|---|
| Calculé | C% 65,13 | H% 4,65 | Cl% 9,61 | N% 7,60 | O% 13,01 |
| Trouvé | 64,99 | 4,75 | 9,63 | 7,58 | 13,05 |

Spectre RMN (CDCl₃) :
1,13-1,34 (4H,m) ; 2,1-2,22 (1H,m) ; 2,22 (3H,s) ; 6,83 (2H,d, J=9,2) ; 6,89 (1H,d, J=8,8) ; 7,23-7,30 (3H,m) ; 7,36 (1H,d, J=2,6) ; 7,47 (1H,s) ; 15,84 (1H,s).
Spectre IR :
3270 (m), 2180 (m), 1600 (m), 1520 (s), 1470 (s), 1400 (s), 1330 (m), 1240 (m), 1200 (s), 1180 (m), 1070 (m), 995 (m), 880 (m), 830 (m), 790 (m).

### Exemple 13 : 2-cyano 3-cyclopropyl N-[4-(4'-chlorophénoxy) 2-méthylphényl] 3-hydroxy prop-2-énamide.

Rendt. 66%.
F = 144,0°-145,0°C.

| Analyse : C₂₀H₁₇ClN₂O₃ = 368,82 | | | | | |
|---|---|---|---|---|---|
| Calculé | C% 65,13 | H% 4,65 | Cl% 9,61 | N% 7,60 | O% 13,01 |
| Trouvé | 65,07 | 4,71 | 9,67 | 7,48 | 13,07 |

Spectre RMN (CDCl₃) :
1,13-1,34 (4H,m) ; 2,1-2,22 (1H,m) ; 2,27 (3H,s) ; 6,84-6,97 (4H,m) ; 7,28-7,54 (4H,m) ; 15,86 (1H,s).
Spectre IR :
3260 (m), 2215 (m), 1580 (s), 1540 (s), 1480 (s), 1415 (s), 1350 (m), 1290 (s), 1255 (m), 1225 (s), 1200 (s), 1165 (m), 1080 (m), 1000 (m), 950 (m), 900 (m), 875 (m), 825 (s), 810 (s), 655 (m).

### Exemple 14 : 2-cyano 3-cyclopropyl N-[4-(4'-bromophénoxy) phényl] 3-hydroxy prop-2-énamide.

Rendt. 74%.
F = 154,0°-155,0°C.

| Analyse : C₁₉H₁₅BrN₂O₃ = 399,25 | | | | | |
|---|---|---|---|---|---|
| Calculé | C% 57,16 | H% 3,79 | Br% 20,01 | N% 7,02 | O% 12,02 |
| Trouvé | 57,20 | 3,90 | 19,74 | 6,95 | 12,21 |

Spectre RMN (CDCl₃) :
1,13-1,36 (4H,m) ; 2,09-2,21 (1H,m) ; 6,90 (2H,d, J=10,4) ; 7,01 (2H,d, J=10) ; 7,4-7,48 (4H,m) ; 7,56 (1H,s) ; 15,85 (1H,s).
Spectre IR :
3260 (m), 2250 (m), 1600 (m), 1570 (s), 1540 (s), 1500 (s), 1480 (s), 1420 (m), 1350 (m), 1270 (m), 1255 (s), 1230 (s), 1190 (m), 1160 (m), 1000 (m), 875 (m), 820 (s).

### Exemple 15 : 2-cyano 3-cyclopropyl N-[4-(4′-cyanophénoxy) phényl] 3-hydroxy prop-2-énamide.

Rendt. 87%.
F = 180,0°-182,0°C.

| Analyse : C₂₀H₁₅N₃O₃ = 345,36 | | | | |
|---|---|---|---|---|
| Calculé | C% 69,56 | H% 4,38 | N% 12,17 | O% 13,90 |
| Trouvé | 69,83 | 4,56 | 11,99 | 13,62 |

Spectre RMN (CDCl₃) :
1,12-1,38 (4H,m) ; 2,1-2,22 (1H,m) ; 7,02 (2H,d, J=9) ; 7,08 (2H,d, J=8,8) ; 7,49-7,65 (5H,m) ; 15,76 (1H,s).
Spectre IR :
3260 (m), 2210 (m), 1595 (s), 1570 (m), 1540 (s), 1495 (s), 1415 (m), 1350 (m), 1285 (m), 1255 (s), 1230 (s), 1190 (m), 1165 (m), 875 (m), 830 (m).

### Exemple 16 : 2-cyano 3-cyclopropyl 3-hydroxy N-[4-(4′-trifluorométhoxyphénoxy) phényl] prop-2-énamide.

Rendt. 65%.
F = 126,0°-127,0°C.

| Analyse : C₂₀H₁₅F₃N₂O₄ = 404,35 | | | | | |
|---|---|---|---|---|---|
| Calculé | C% 59,41 | H% 3,74 | F% 14,10 | N% 6,93 | O% 15,83 |
| Trouvé | 59,31 | 3,79 | 14,14 | 6,89 | 15,87 |

Spectre RMN (CDCl₃) :
1,15-1,33 (4H,m) ; 2,11-2,19 (1H,m) ; 6,99 (2H,d, J=3,94) ; 7,03 (2H,d, J=3,78) ; 7,19 (2H,d) ; 7,45 (2H,d J=8,94) ; 7,54 (1H,s) ; 15,86 (1H,s).
Spectre IR :
3320 (m), 2190 (m), 1595 (m), 1575 (m), 1535 (s), 1480 (s), 1400 (m), 1340 (m), 1250 (s), 1235 (s), 1220 (s), 1205 (m), 1180 (s), 1150 (s), 880 (m), 825 (m).

### Exemple 17 : 2-cyano 3-cyclopropyl N-[4-(4′-t-butylphénoxy) phényl] 3-hydroxy prop-2-énamide.

Rendt. 39%.
F = 125,0°-126,0°C.

| Analyse : C₂₃H₂₄N₂O₃ = 376,46 | | | | |
|---|---|---|---|---|
| Calculé | C% 73,38 | H% 6,43 | N% 7,44 | O% 12,75 |
| Trouvé | 73,13 | 6,64 | 7,14 | 13,09 |

Spectre RMN (CDCl₃) :
1,10-1,46 (13H,m) ; 2,11-2,19 (1H,s) ; 6,94 (2H,d, J=8,60) ; 7,00 (2H,d, J=9,0) ; 7,36 (2H,d, J=9,0) ; 7,40 (2H,d J=9,0) ; 7,52 (1H,s) ; 15,92 (1H,s).
Spectre IR :
3340 (m), 3280 (m), 2960 (m), 2860 (m), 2200 (s), 1580 (s), 1545 (s), 1495 (s), 1410 (s), 1350 (s), 1310 (m), 1285 (m), 1245 (s), 1220 (s), 1170 (m), 1105 (m), 1055 (m), 1005 (m), 890 (m), 825 (m).

### Exemple 18 : 2-cyano 3-cyclopropyl 3-hydroxy N-[4-(4′-méthylphénoxy) phényl] prop-2-énamide.

Rendt. 65%.
F = 146,0°-147,0°C.

| Analyse : C₂₀H₁₈N₂O₃ = 334,38 | | | | |
|---|---|---|---|---|
| Calculé | C% 71,84 | H% 5,43 | N% 8,38 | O% 14,35 |
| Trouvé | 71,93 | 5,54 | 8,30 | 14,23 |

Spectre RMN (CDCl₃) :
1,09-1,35 (4H,m) ; 2,08-2,21 (1H,m) ; 2,34 (3H,s) ; 6,91 (2H,d, J=8,6) ; 6,97 (2H,d, J=9,0) ; 7,15 (2H,d, J=8,4) ; 7,39 (2H,d J=8,8) ; 7,51 (1H,s) ; 15,92 (1H,s).
Spectre IR :
3260 (m), 2210 (m), 1595 (s), 1580 (s), 1530 (s), 1495 (s), 1420 (s), 1345 (s), 1305 (m), 1250 (s), 1225 (s), 1165 (m), 985 (m), 895 (m), 875 (m), 820 (m), 685 (m).

### Exemple 19 : 2-cyano 3-cyclopropyl 3-hydroxy N-[4-(4′-méthoxyphénoxy) phényl] prop-2-énamide.

Rendt. 57%.
F = 139,0°-140,0°C.
Spectre RMN CDCl₃ :
1,10-1,36 (4H,m) ; 2,11-2,19 (1H,m) ; 6,87-7,01 (6H,m) ; 7,38 (2H,d, J=9,0) ; 7,53 (1H,s) ; 15,94 (1H,s).
Spectre IR :
3280 (s), 2200 (s), 1610 (m), 1590 (m), 1560 (s), 1520 (s), 1490 (s), 1460 (m), 1435 (m), 1410 (m), 1340 (m), 1240 (s), 1210 (s), 1170 (m), 1020 (m), 885 (m), 830 (m), 815 (m).

### Exemple 20 : 2-cyano 3-cyclobutyl N-[4-(4′-fluorophénoxy) phényl] 3-hydroxy prop-2-énamide.

Rendt. 81%.
F = 143,0°-144,0°C.

| Analyse : C₂₀H₁₇FN₂O₃ = 352,37 | | | | | |
|---|---|---|---|---|---|
| Calculé | C% 68,17 | H% 4,86 | F% 5,39 | N% 7,95 | O% 13,62 |
| Trouvé | 68,05 | 4,97 | 5,40 | 7,90 | 13,68 |

Spectre RMN (CDCl₃) :
1,91-2,49 (6H,m) ; 3,65 (1H,q J=8,4) ; 6,93-7,11 (6H,m) ; 7,41 (2H,d, J=9,0) ; 7,53 (1H,s) ; 15,82 (1H,s).
Spectre IR :
3270 (s), 2980 (m), 2940 (m), 2220 (m), 1610 (s), 1575 (s), 1545 (s), 1490 (s), 1440 (m), 1415 (m), 1385 (m), 1325 (m), 1240 (m), 1205 (s), 820 (s).

### Exemple 21 : 2-cyano 3-cyclopentyl N-[4-(4′-fluorophénoxy) phényl] 3-hydroxy prop-2-énamide.

Rendt. 44%.
F = 119,0°-120,0°C.

| Analyse : C₂₁H₁₉FN₂O₃ = 366,40 | | | | | |
|---|---|---|---|---|---|
| Calculé | C% 68,84 | H% 5,23 | F% 5,19 | N% 7,65 | O% 13,10 |
| Trouvé | 68,86 | 5,36 | 5,17 | 7,66 | 12,95 |

Spectre RMN (CDCl₃) :
1,59-2,05 (8H,m) ; 3,17-3,24 (1H,m) ; 6,93-7,13 (6H,m) ; 7,41 (2H,d, J=9,0) ; 7,56 (1H,s) ; 15,76 (1H,s).
Spectre IR :
3280 (s), 2950 (m), 2870 (m), 2205 (s), 1590 (s), 1535 (s), 1495 (s), 1420 (m), 1390 (m), 1350 (m), 1300 (m), 1250 (s), 1215 (s), 1190 (s), 1165 (m), 1095 (m), 995 (m), 850 (m), 825 (s), 805 (m).

### Exemple 22 : 2-cyano 3-cyclopropyl N-[4′-(chlorophénylthio) phényl] 3-hydroxy prop-2-énamide.

Rendt. 81%.
F = 147,5°-148,0°C.

| Analyse : C₁₉H₁₅ClN₂O₂S = 370,86 | | | | | | |
|---|---|---|---|---|---|---|
| Calculé | C% 61,54 | H% 4,08 | Cl% 9,56 | N% 7,55 | O% 8,63 | S% 8,65 |
| Trouvé | 61,51 | 4,23 | 9,57 | 7,48 | 8,64 | 8,57 |

Spectre RMN (CDCl₃) :
1,10-1,22 (2H,m) ; 1,24-1,36 (2H,m) ; 2,09-2,19 (1H,m) ; 7,17-7,27 (4H,m) ; 7,35 (2H,d J=8,76) ; 7,46 (2H,d, J=8,72) ; 7,54 (1H,s) ; 15,73 (1H,s).
Spectre IR :
3466 (m), 3394 (m), 3058 (m), 2220 (m), 1900 (m), 1668 (m), 1622 (m), 1578 (s), 1530 (s), 1489 (m), 1469 (m), 1453 (m), 1402 (m), 1375 (m), 1345 (m), 1330 (m), 1310 (m), 1260 (m), 1229 (m), 1116 (m), 1100 (m), 1084 (m), 1006 (m), 983 (m).

### Exemple 23 : 2-cyano 3-cyclopropyl N-[4'-(chlorophénylsulphonyl) phényl] 3-hydroxy prop-2-énamide.

Rendt. 85%.
F = 210,0°-212,0°C.

| Analyse : C₁₉H₁₅ClN₂O₄S = 402,86 | | | | | | |
|---|---|---|---|---|---|---|
| Calculé | C% 56,65 | H% 3,75 | Cl% 8,80 | N% 6,95 | O% 15,89 | S% 7,96 |
| Trouvé | 56,52 | 3,85 | 8,75 | 6,96 | 16,06 | 7,86 |

Spectre RMN (CDCl₃) :
1,14-1,27 (2H,m) ; 1,31-1,39 (2H,m) ; 2,08-2,21 (1H,m) ; 7,48 (2H,d J=8,8) ; 7,67 (2H,d, J=8,8) ; 7,75 (1H,s) ; 7,87 (2H,d J=8,6) ; 7,92 (2H,d J=8,6) ; 15,42 (1H,s).
Spectre IR :
3286 (m), 2216 (m), 1575 (s), 1569 (s), 1533 (s), 1496 (m), 1404 (m), 1350 (m), 1317 (m), 1310 (m), 1262 (m), 1150 (s), 1104 (m), 1087 (m), 993 (m), 837 (m), 757 (s), 707 (m), 653 (m).

### Exemple 24 :

On a préparé des comprimés répondant à la formule suivante :

| | |
|---|---|
| - Composé de l'exemple 4 | 20 mg |
| - Excipient q.s.p. un comprimé terminé à | 150 mg |
| (Détail de l'excipient : lactose, amidon, talc, stéarate de magnésium). | |

### Exemple 25 :

On a préparé des comprimés répondant à la formule suivante :

| | |
|---|---|
| - Composé de l'exemple 1 | 20 mg |
| - Excipient q.s.p. un comprimé terminé à | 150 mg |
| (Détail de l'excipient : lactose, amidon, talc, stéarate de magnésium). | |

### ACTIVITE PHARMACOLOGIQUE

### Méthodes de tests biochimiques.

### 1er test

Oedème de la patte de rat (PO-R) induit par de la carragénine.

Une heure après l'administration orale de 50 mg/kg des composés du test ou du véhicule témoin à des groupes de rats (n = 6-12, CFHB mâles, d'un poids entre 160-180 g), on injecte 1 mg de carragénine dissoute dans 0,2 ml de solution saline dans le coussinet digital de la patte arrière droite. Les pattes contralatérales reçoivent les injections témoin de solution saline. Les réactions d'oedèmes des pattes sont évaluées trois heures plus tard.

### 2ème test

Oedème à hypersensibilité de type retard de la patte de souris (DTH-M).

Des groupes de souris (n = 8-10), mâles CD-1, d'un poids entre 25-30 g) sont sensibilisés par une injection sous-cutanée de 1 mg de sérum-albumine bovine méthylé (MBSA) dans des volumes de 0,2 ml d'une solution saline/émulsion d'adjuvant complet de Freund (FCA). Des groupes témoin négatifs reçoivent des injections de solution saline/émulsion de FCA. Les réactions DHT d'oedème de la patte sont évaluées 24 heures après le défi dans le coussinet digital de la patte arrière droite avec 0,1 mg de MBSA dans des volumes de 0,05 ml de solution saline le 7ème jour après la sensibilisation. Les pattes contralatérales reçoivent des injections témoin de solution saline. Les composés du test ou les véhivules témoin sont administrés oralement une fois par jour les 4ème, 5ème et 6ème jours et deux fois par jour le 7ème jour, 1 heure avant et 6 heures après le défi au MBSA.

### 3ème test

Oedème à hypersensibilité de type retard de la patte de rat (DTH-R).

Des groupes de rats (n = 8-12, mâles CFHB, d'un poids entre 160-180 g) sont sensibilisés par une injection sous-cutanée à la base de la queue avec des volumes de 0,1 ml de FCA. Des groupes témoin négatifs reçoivent une injection d'adjuvant incomplet de Freund. Les réactions DTH d'oedème de la patte sont évaluées 24 heures après le défi dans le coussinet digital de la patte arrière droite avec 0,4 mg d'un extrait antigène de Mycobacterium tuberculosis dans des volumes de 0,2 ml de solution saline le 7ème jour après la sensibilisation. Les pattes contralatérales reçoivent des injections témoin de solution saline.

Les composés du test sont administrés oralement une fois par jour les 4ème, 5ème et 6ème jour et deux fois par jour le 7ème jour, 1 heure avant et 6 heures après le défi anti-génique.

Les résultats de ces tests sont donnés au tableau II.

Les doses sont données en unités de mg/kg p.o.

**TABLEAU**

| Exemple | Test 1 | Test 2 | Test 3 |
|---|---|---|---|
| 1 | 25 | 63 (30) | 31(10) |
| 2 | 20 | 9(100) | 6(50) |
| 3 | 14 | 66(100) | 22(50) |
| 4 | 23 | 104(100) | 44(50) |
| 5 | 25 | 18 (30) | 59(10) |
| 6 | 24 | 86 (30) | 38(10) |
| 7 | 18 | 16 (30) | 12(10) |
| 8 | 29 (50) | -17 (30) | 39 (10) |
| 9 | 20 (50) | -18 (100) | 27 (50) |
| 10 | 24 (50) | 2 (100) | 47 (50) |
| 11 | 20 (50) | 11 (100) | 66 (50) |
| 12 | 2 (50) | 53 (30) | 50 (10) |
| 13 | 3 (10) | 49 (30) | 13 (10) |
| 14 | 14 (10) | 42 (30) | 38 (10) |
| 15 | 10 (10) | 61 (100) | 29 (10) |
| 16 | 15 (10) | 21 (10) | 64 (10) |
| 17 | 20 (50) | 25 (100) | 43 (50) |
| 18 | 16 (50) | 47 (100) | 30 (50) |
| 19 | 7 (50) | 12 (100) | -1 (50) |
| 20 | 14 (50) | -5 (30) | 31 (50) |
| 21 | 3 (50) | 28 (30) | 22 (50) |
| 22 | 17 (50) | 62 (100) | 25 (50) |
| 23 | 15 (50) | 11 (100) | -13 (50) |

## Revendications (Revendications pour l'(les) Etat(s) contractant(s) suivant(s): AT, BE, CH, DE, DK, FR, GB, IE, IT, LI, LU, NL, PT, SE)

1. Nouveaux dérivés du 3-cycloalkyl-propen-2-amide repondant à la formule générale (I) : 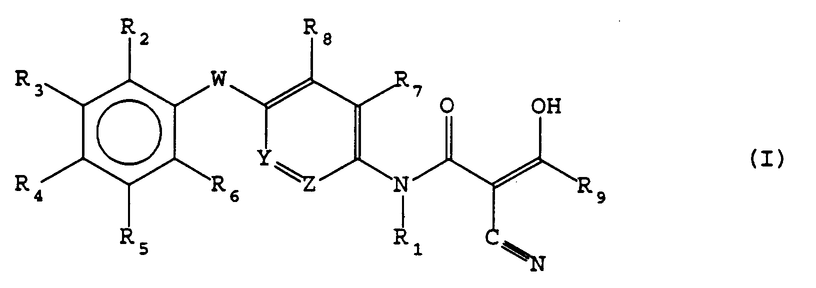 dans laquelle :
- R₁ représente un atome d'hydrogène ou un radical alkyle renfermant de 1 à 3 atomes de carbone,
- W représente un atome d'oxygène ou de soufre éventuellement oxydé sous forme de sulfoxyde ou de sulfone,
- Y et Z représentent un groupement -CH- ou l'un de Y et Z représente un groupement -CH- tandis que l'autre représente un atome d'azote,
- R₂, R₃, R₄, R₅ et R₆, identiques ou différents, représentent un atome d'hydrogène, un atome d'halogène, un radical alkyle renfermant de 1 à 4 atomes de carbone, un radical méthoxy, un radical méthylthio, un radical -WCF₃ dans lequel W a la signification indiquée ci-dessus, un groupement -CF₃, un groupement NO₂, un groupement nitrile, un groupement -W(CH₂)ₙ-CF₃, -W(CF₂)ₙ-CF₃ et (CF₂)ₙ-CF₃ dans lequel W a la signification déja indiquée et n représente un nombre entier égal à 1, 2 ou 3 , un groupement -(CH₂)ₙ-CX₃ dans lequel n a la signification déja indiquée et X représente un atome d'halogène, ou R₃ et R₄ forment ensemble un groupement -O-CH₂-O- et R₂, R₅, et R₆ ont la signification indiquée ci-dessus,
- R₇ et R₈, identiques ou différents représentent un atome d'hydrogène ou un radical alkyle renfermant de 1 à 6 atomes de carbone,
- R₉ représente un radical cycloalkyle renfermant de 3 à 6 atomes de carbone,
leurs formes tautomères, ainsi que leurs sels d'addition avec les bases minérales ou organiques.

2. Nouveaux dérivés du cycloalkyl-propen-2-amide tels que définis par la formule (I) de la revendication 1, caractérisés en ce que dans ladite formule (I),
- R_{**1**} représente un atome d'hydrogène ou un radical méthyle,
- W représente un atome d'oxygène,
- R_{**2**}, R_{**3**}, R_{**4**}, R_{**5**} et R_{**6**}, identiques ou différents, représentent un atome d'hydrogène, un atome de fluor, de chlore, de brome ou d'iode, un radical méthyle, un radical méthoxy, un radical -OCF_{**3**} , un radical -CF_{**3**}, un groupement NO_{**2**}, un groupement nitrile, un groupement -W(CH_{**2**})_{**n**}-CF_{**3**} dans lequel W et n ont la signification déja indiquée,
ou, R_{**3**} et R_{**4**} forment ensemble un groupement -O-CH_{**2**}-O-,
et R_{**2**}, R_{**5**}, et R_{**6**} représentent un atome d'hydrogène,
- R_{**7**} et R_{**8**} représentent un atome d'hydrogène,
- R_{**9**} représente un radical cyclopropyle,
- Y et Z ont la signification déja indiquée,
leurs formes tautomères, ainsi que leurs sels d'addition avec les bases minérales ou organiques.

3. Nouveaux dérivés du 3-cycloalkyl-propèn-2-amide répondant à la formule (I) selon la revendication 1 ou 2, caractérisés en ce que dans ladite formule (I), R_{**1**} représente un atome d'hydrogène, W représente un atome d'oxygène, R_{**2**}, R_{**3**}, R_{**4**}, R_{**5**} et R_{**6**} , identiques ou diffé-rents, représentent un atome d'hydrogène, un atome de fluor, de chlore, d'iode ou un groupement NO_{**2**}, ou, R_{**3**} et R_{**4**} forment ensemble un groupement -O-CH_{**2**}-O-, et R_{**2**}, R_{**5**} et R_{**6**} représentent un atome d'hydrogène, R_{**7**} et R_{**8**} représentent un atome d'hydrogène et R_{**9**} représente un radical cyclopropyle et Y et Z ont la signification déja indiquée, leurs formes tautomères, ainsi que leurs sels d'addition avec les bases minérales ou organiques.

4. Le N-[4-(4′-chlorophénoxy)phényl]-2-cyano-3-cyclopropyl-3-hydroxy prop-2-enamide, ainsi que ses sels d'addition avec les bases minérales ou organiques.

5. Le 2-cyano-3-cyclopropyl-3-hydroxy-N-[4-(4′-nitrophénoxy) phényl] prop-2-enamide, ainsi que ses sels d'addition avec les bases minérales ou organiques.

6. Le 2-cyano-3-cyclopropyl-3-hydroxy-N-[4-(3′,4′-methylene-dioxyphénoxy)phenyl] prop-2-enamide, ainsi que ses sels d'addition avec les bases minérales ou organiques.

7. Le 2-cyano-3-cyclopropyl-N-[4-(4′-fluorophénoxy)phenyl] 3-hydroxy prop-2-enamide, ainsi que ses sels d'addition avec les bases minérales ou organiques.

8. Le 2-cyano-3-cyclopropyl-3-hydroxy-N-[4-(4′-iodophénoxy)phenyl] prop-2-enamide, ainsi que ses sels d'addition avec les bases minérales ou organiques.

9. Procédé de préparation des nouveaux dérivés du 3-cycloalkyl-propen-2-amide tels que définis par la formule (I) de la revendication 1, ainsi que de leurs sels d'addition avec les bases minérales ou organiques, caractérisé en ce que l'on fait réagir un produit de formule (II) : 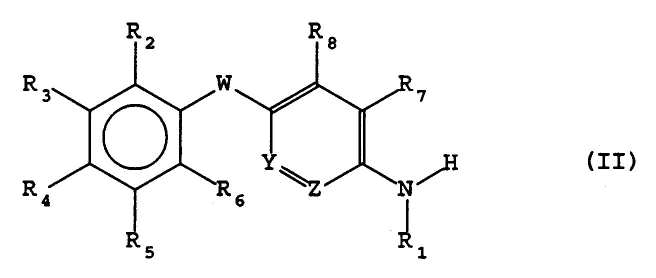 dans laquelle R_{**1**}, R_{**2**}, R_{**3**}, R_{**4**}, R_{**5**}, R_{**6**}, R_{**7**}, R_{**8**}**,** W, Y et Z ont la signification déja indiquée, avec un produit de formule (III):
HO-CO-CH_{**2**}-CN (III)
pour obtenir un produit de formule (IV) : 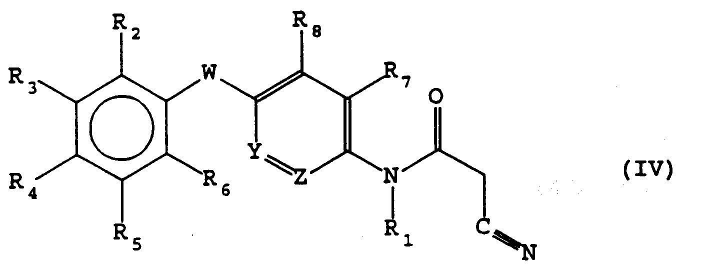 dans laquelle R_{**1**}, R_{**2**}, R_{**3**}, R_{**4**}, R_{**5**}, R_{**6**}, R_{**7**}, R_{**8**}, W, Y et Z ont la signification déja indiquée, puis, fait réagir ce dernier, successivement avec de l'hydrure de sodium, puis avec un produit de formule (V):
Hal-CO-R_{**9**} (V)
dans laquelle Hal représente un atome d'halogène et R_{**9**} a la signification déjà indiquée, pour obtenir le produit de formule (I) recherché, que l'on peut salifier si désiré.

10. Procédé selon la revendication 9, caractérisé en ce que :
- la réaction du produit de formule (II) avec le produit de formule (III) est effectuée en présence de pentachlorure de phosphore au sein d'un solvant organique anhydre, tel que le chlorure de méthylène ou le tétrahydrofurane,
- la réaction du produit de formule (IV) avec l'hydrure de sodium est éffectuée au sein d'un solvant organique anhydre tel que le tétrahydrofurane, le cas échéant en présence d'un catalyseur tel qu'un imidazole.

11. Médicaments caractérisés en ce qu'ils sont constitués par les nouveaux dérivés du 3-cycloalkyl-propen-2-amide, tels que définis par la formule (I) de la revendication 1, ainsi que par leurs sels d'addition avec les bases minérales ou organiques pharmaceutiquement acceptables.

12. Médicaments caractérisés en ce qu'ils sont constitués par les nouveaux dérivés du 3-cycloalkyl-propen-2-amide, tels que définis à l'une quelconque des revendications 2 à 8, ainsi que par leurs sels d'addition avec les bases minérales ou organiques pharmaceutiquement acceptables.

13. Compositions pharmaceutiques, caractérisées en ce qu'elles renferment à titre de principe actif l'un au moins des médicaments tels que définis à l'une quelconque des revendications 11 et 12.

14. A titre de produits industriels nouveaux, les produits de formule (IV) : 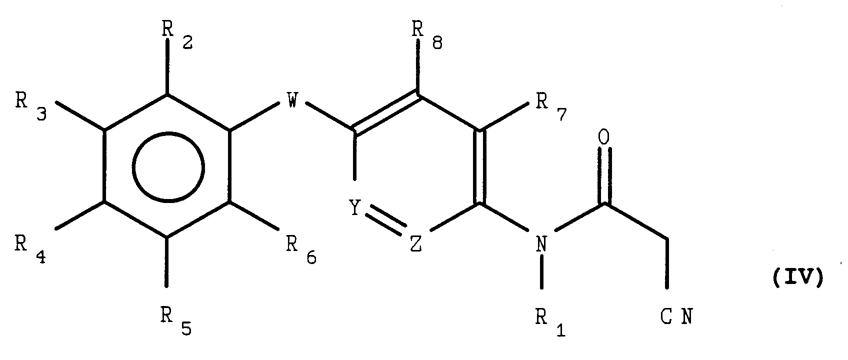 dans laquelle
R₁, R₂, R₃, R₄, R₅, R₆, R₇, R₈ et W ont la signification déjà indiquée et l'un de Y et Z représente un groupement -CH-tandis que l'autre représente un atome d'azote.

## Revendications (Revendications pour l'(les) Etat(s) contractant(s) suivant(s): ES)

1. Procédé pour préparer des nouveaux dérivés du 3-cycloalkyl-propen-2-amide répondant à la formule générale (I) : 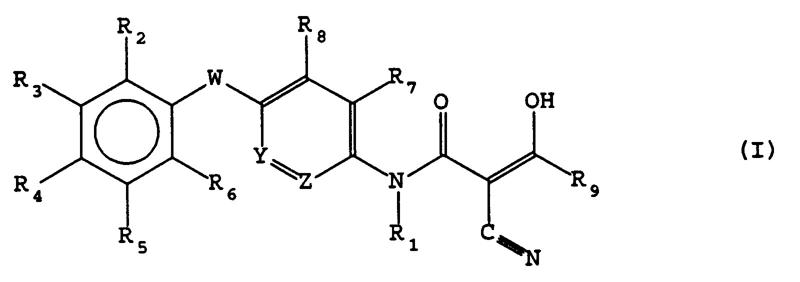 dans laquelle :
- R_{**1**} représente un atome d'hydrogène ou un radical alkyle renfermant de 1 à 3 atomes de carbone,
- W représente un atome d'oxygène ou de soufre éventuellement oxydé sous forme de sulfoxyde ou de sulfone,
- Y et Z représentent un groupement -CH- ou l'un de Y et Z représente un groupement -CH- tandis que l'autre représente un atome d'azote,
- R_{**2**}, R_{**3**}, R_{**4**}, R_{**5**} et R_{**6**}, identiques ou différents, représentent un atome d'hydrogène, un atome d'halogène, un radical alkyle renfermant de 1 à 4 atomes de carbone, un radical méthoxy, un radical méthylthio, un radical -WCF_{**3**} dans lequel W a la signification indiquée ci-dessus, un groupement -CF_{**3**}, un groupement NO_{**2**}, un groupement nitrile, un groupement -W(CH_{**2**})_{**n**}-CF_{**3**}, -W(CF_{**2**})_{**n**}-CF_{**3**} et (CF_{**2**})_{**n**}-CF_{**3**} dans lequel W a la signification déja indiquée et n représente un nombre entier égal à 1, 2 ou 3 , un groupement -(CH_{**2**})_{**n**}-CX_{**3**} dans lequel n a la signification déja indiquée et X représente un atome d'halogène, ou R_{**3**} et R_{**4**} forment ensemble un groupement -O-CH_{**2**}-O- et R_{**2**}, R_{**5**}, et R_{**6**} ont la signification indiquée ci-dessus,
- R_{**7**} et R_{**8**}, identiques ou différents représentent un atome d'hydrogène ou un radical alkyle renfermant de 1 à 6 atomes de carbone,
- R_{**9**} représente un radical cycloalkyle renfermant de 3 à 6 atomes de carbone,
leurs formes tautomères, ainsi que leurs sels d'addition avec les bases minérales ou organiques, caractérisé en ce que l'on fait réagir un produit de formule (II) : 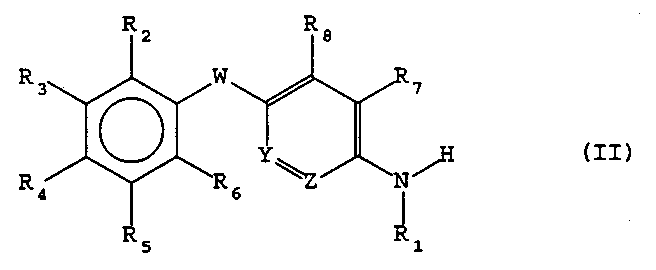 dans laquelle R_{**1**}, R_{**2**}, R_{**3**}, R_{**4**}, R_{**5**}, R_{**6**}, R_{**7**}, R_{**8**}, W, Y et Z ont la signification déja indiquée, avec un produit de formule (III):
HO-CO-CH_{**2**}-CN (III)
pour obtenir un produit de formule (IV) : 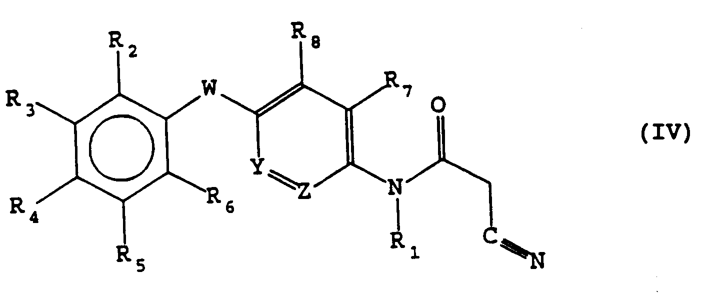 dans laquelle R_{**1**}, R_{**2**}, R_{**3**}, R_{**4**}, R_{**5**}, R_{**6**}, R_{**7**}, R_{**8**}, W, Y et Z ont la signification déja indiquée, puis, fait réagir ce dernier, successivement avec de l'hydrure de sodium, puis avec un produit de formule (V):
Hal-CO-R_{**9**} (V)
dans laquelle Hal représente un atome d'halogène et R_{**9**} a la signification déjà indiquée, pour obtenir le produit de formule (I) recherché, que l'on peut salifier si désiré.

2. Procédé selon la revendication 1, caractérisé en ce que :
- la réaction du produit de formule (II) avec le produit de formule (III) est effectuée en présence de pentachlorure de phosphore au sein d'un solvant organique anhydre, tel que le chlorure de méthylène ou le tétrahydrofurane,
- la réaction du produit de formule (IV) avec l'hydrure de sodium est éffectuée au sein d'un solvant organique anhydre tel que le tétrahydrofurane, le cas échéant en présence d'un catalyseur tel qu'un imidazole.

3. Procédé selon la revendication 1 ou 2, caractérisé en ce que l'on utilise au départ un produit de formule (II) dans laquelle :
- R_{**1**} représente un atome d'hydrogène ou un radical méthyle,
- W représente un atome d'oxygène,
- R_{**2**}, R_{**3**}, R_{**4**}, R_{**5**} et R_{**6**}, identiques ou différents, représentent un atome d'hydrogène, un atome de fluor, de chlore, de brome ou d'iode, un radical méthyle, un radical méthoxy, un radical -OCF_{**3**} , un radical -CF_{**3**}, un groupement NO_{**2**}, un groupement nitrile, un groupement -W(CH_{**2**})_{**n**}-CF_{**3**} dans lequel W et n ont la signification déja indiquée,
ou, R_{**3**} et R_{**4**} forment ensemble un groupement -O-CH_{**2**}-O-, et R_{**2**}, R_{**5**}, et R_{**6**} représentent un atome d'hydrogène,
- R_{**7**} et R_{**8**} représentent un atome d'hydrogène,
- Y et Z ont la signification déja indiquée,
et un produit de formule (V) dans laquelle R_{**9**} représente un radical cyclopropyle.

4. Procédé selon l'une quelconque des revendications 1 à 3, caractérisé en ce que l'on utilise au départ un produit de formule (II) dans laquelle :
R_{**1**} représente un atome d'hydrogène, W représente un atome d'oxygène, R_{**2**}, R_{**3**}, R_{**4**}, R_{**5**} et R_{**6**}, identiques ou diffé-rents, représentent un atome d'hydrogène, un atome de fluor, de chlore, d'iode ou un groupement NO_{**2**}, ou, R_{**3**} et R_{**4**} forment ensemble un groupement -O-CH_{**2**}-O-, et R_{**2**}, R_{**5**} et R_{**6**} représentent un atome d'hydrogène, R_{**7**} et R_{**8**} représentent un atome d'hydrogène et Y et Z ont la signification déja indiquée, et un produit de formule (V) dans laquelle R_{**9**} représente un radical cyclopropyle.

## Revendications (Revendications pour l'(les) Etat(s) contractant(s) suivant(s): GR)

1. Procédé pour préparer des nouveaux dérivés du 3-cycloalkyl-propen-2-amide répondant à la formule générale (I) : 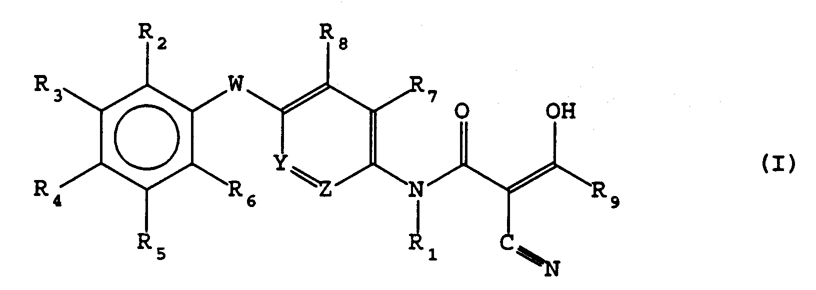 dans laquelle :
- R_{**1**} représente un atome d'hydrogène ou un radical alkyle renfermant de 1 à 3 atomes de carbone,
- W représente un atome d'oxygène ou de soufre éventuellement oxydé sous forme de sulfoxyde ou de sulfone,
- Y et Z représentent un groupement -CH- ou l'un de Y et Z représente un groupement -CH- tandis que l'autre représente un atome d'azote,
- R_{**2**}, R_{**3**}, R_{**4**}, R_{**5**} et R_{**6**}, identiques ou différents, représentent un atome d'hydrogène, un atome d'halogène, un radical alkyle renfermant de 1 à 4 atomes de carbone, un radical méthoxy, un radical méthylthio, un radical -WCF_{**3**} dans lequel W a la signification indiquée ci-dessus, un groupement -CF_{**3**}, un groupement NO_{**2**}, un groupement nitrile, un groupement -W(CH_{**2**})_{**n**}-CF_{**3**}, -W(CF_{**2**})_{**n**}-CF_{**3**} et (CF_{**2**})_{**n**}-CF_{**3**} dans lequel W a la signification déja indiquée et n représente un nombre entier égal à 1, 2 ou 3 , un groupement -(CH_{**2**})_{**n**}-CX_{**3**} dans lequel n a la signification déja indiquée et X représente un atome d'halogène, ou R_{**3**} et R_{**4**} forment ensemble un groupement -O-CH_{**2**}-O- et R_{**2**}, R_{**5**}, et R_{**6**} ont la signification indiquée ci-dessus,
- R_{**7**} et R_{**8**}, identiques ou différents représentent un atome d'hydrogène ou un radical alkyle renfermant de 1 à 6 atomes de carbone,
- R_{**9**} représente un radical cycloalkyle renfermant de 3 à 6 atomes de carbone,
leurs formes tautomères, ainsi que leurs sels d'addition avec les bases minérales ou organiques, caractérisé en ce que l'on fait réagir un produit de formule (II) : 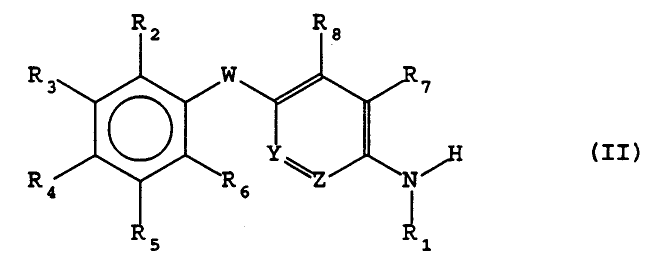 dans laquelle R_{**1**}, R_{**2**}, R_{**3**}, R_{**4**}, R_{**5**}, R_{**6**}, R_{**7**}, R_{**8**}, W, Y et Z ont la signification déja indiquée, avec un produit de formule (III):
HO-CO-CH_{**2**}-CN (III)
pour obtenir un produit de formule (IV) : 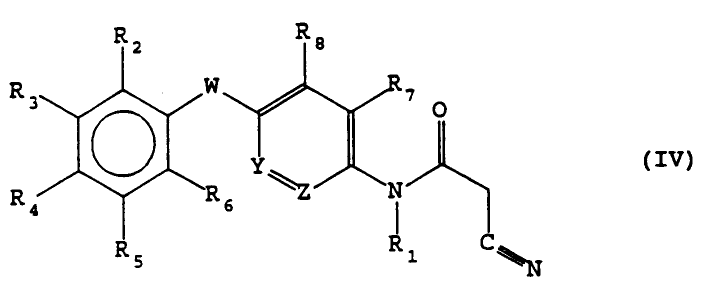 dans laquelle R_{**1**}, R_{**2**}, R_{**3**}, R_{**4**}, R_{**5**}, R_{**6**}, R_{**7**}, R_{**8**}, W, Y et Z ont la signification déja indiquée, puis, fait réagir ce dernier, successivement avec de l'hydrure de sodium, puis avec un produit de formule (V):
Hal-CO-R_{**9**} (V)
dans laquelle Hal représente un atome d'halogène et R_{**9**} a la signification déjà indiquée, pour obtenir le produit de formule (I) recherché, que l'on peut salifier si désiré.

2. Procédé selon la revendication 1, caractérisé en ce que :
- la réaction du produit de formule (II) avec le produit de formule (III) est effectuée en présence de pentachlorure de phosphore au sein d'un solvant organique anhydre, tel que le chlorure de méthylène ou le tétrahydrofurane,
- la réaction du produit de formule (IV) avec l'hydrure de sodium est éffectuée au sein d'un solvant organique anhydre tel que le tétrahydrofurane, le cas échéant en présence d'un catalyseur tel qu'un imidazole.

3. Procédé selon la revendication 1 ou 2, caractérisé en ce que l'on utilise au départ un produit de formule (II) dans laquelle :
- R_{**1**} représente un atome d'hydrogène ou un radical méthyle,
- W représente un atome d'oxygène,
- R_{**2**}, R_{**3**}, R_{**4**}, R_{**5**} et R_{**6**}, identiques ou différents, représentent un atome d'hydrogène, un atome de fluor, de chlore, de brome ou d'iode, un radical méthyle, un radical méthoxy, un radical -OCF_{**3**} , un radical -CF_{**3**}, un groupement NO_{**2**}, un groupement nitrile, un groupement -W(CH_{**2**})_{**n**}-CF_{**3**} dans lequel W et n ont la signification déja indiquée,
ou, R_{**3**} et R_{**4**} forment ensemble un groupement -O-CH_{**2**}-O-,
et R_{**2**}, R_{**5**}, et R_{**6**} représentent un atome d'hydrogène,
- R_{**7**} et R_{**8**} représentent un atome d'hydrogène,
- Y et Z ont la signification déja indiquée,
et un produit de formule (V) dans laquelle R_{**9**} représente un radical cyclopropyle.

4. Procédé selon l'une quelconque des revendications 1 à 3, caractérisé en ce que l'on utilise au départ un produit de formule (II) dans laquelle :
R_{**1**} représente un atome d'hydrogène, W représente un atome d'oxygène, R_{**2**}, R_{**3**}, R_{**4**}, R_{**5**} et R_{**6**}, identiques ou diffé-rents, représentent un atome d'hydrogène, un atome de fluor, de chlore, d'iode ou un groupement NO_{**2**}, ou, R_{**3**} et R_{**4**} forment ensemble un groupement -O-CH_{**2**}-O-, et R_{**2**}, R_{**5**} et R_{**6**} représentent un atome d'hydrogène, R_{**7**} et R_{**8**} représentent un atome d'hydrogène et Y et Z ont la signification déja indiquée, et un produit de formule (V) dans laquelle R_{**9**} représente un radical cyclopropyle.

5. Procédé selon l'une quelconque des revendications 1 à 4 caractérisé en ce que l'on utilise au départ un produit de formule (II) et un produit de formule (V) choisis de manière telle que l'on prépare l'un quelconque des produits dont les noms suivent :
- le N-[4-(4′-chlorophénoxy)phenyl]-2-cyano-3-cyclopropyl-3-hydroxy prop-2-enamide, ainsi que ses sels d'addition avec les bases minérales ou organiques,
- le 2-cyano-3-cyclopropyl-3-hydroxy-N-[4-(4′-nitrophéno-xy) - phényl] prop-2-enamide, ainsi que ses sels d'addition avec les bases minérales ou organiques,
- le 2-cyano-3-cyclopropyl-3-hydroxy-N-[4-(3′,4′-methylene-dioxyphénoxy)phényl] prop-2-enamide, ainsi que ses sels d'addition avec les bases minérales ou organiques,
- le 2-cyano-3-cyclopropyl-N-[4-(4′-fluorophénoxy)phényl] 3-hydroxy prop-2-enamide, ainsi que ses sels d'addition avec les bases minérales ou organiques,
- le 2-cyano-3-cyclopropyl-3-hydroxy-N-[4-(4′-iodophénoxy)phenyl] prop-2-enamide, ainsi que ses sels d'addition avec les bases minérales ou organiques.

6. Procédé de préparation de compositions pharmaceutiques, caractérisé en ce que l'on met à titre de principe actif l'un au moins des produits de formule (I) telle que définie à la revendication 1 ou l'un au moins de leurs sels pharmaceutiquement acceptables sous une forme destinée à cet usage.

7. Procédé de préparation de compositions pharmaceutiques, caractérisé en ce que l'on met à titre de principe actif l'un au moins des produits de formule (I) telle que définie à l'une des revendications 2 à 5 ou l'un au moins de leurs sels pharmaceutiquement acceptables sous une forme destinée à cet usage.

8. A titre de produits industriels nouveaux, les produits de formule (IV) : 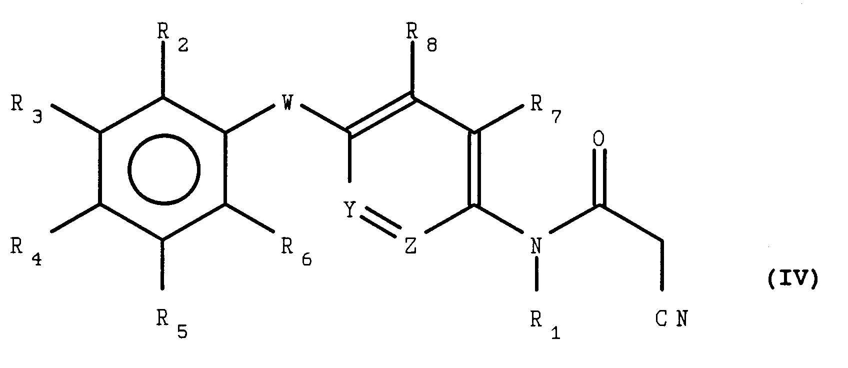 dans laquelle
R₁, R₂, R₃, R₄, R₅, R₆, R₇, R₈ et W ont la signification déjà indiquée et l'un de Y et Z représente un groupement -CH-tandis que l'autre représente un atome d'azote.

## Claims (Claims for the following Contracting State(s): AT, BE, CH, DE, DK, FR, GB, IE, IT, LI, LU, NL, PT, SE)

1. New 3-cycloalkyl-propen-2-amide derivatives corresponding to the general formula (I): 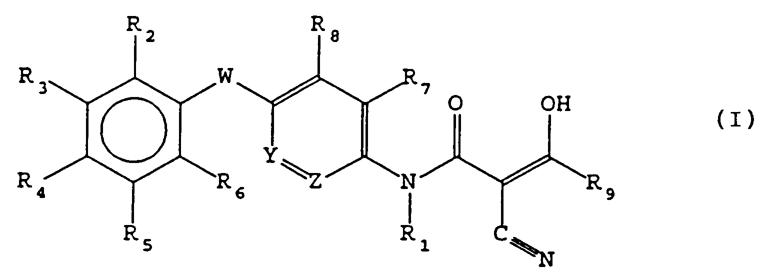 in which:
- R₁ represents a hydrogen atom or an alkyl radical containing 1 to 3 carbon atoms,
- W represents an oxygen or sulphur atom optionally oxidized in the form of the sulphoxide or sulphone,
- Y and Z represent a -CH- group or one of Y and Z represents a -CH- group while the other represents a nitrogen atom,
- R₂, R₃, R₄, R₅ and R₆, identical or different, represent a hydrogen atom, a halogen atom, an alkyl radical containing 1 to 4 carbon atoms, a methoxy radical, a methylthio radical, a -WCF₃ radical in which W has the meaning indicated above, a -CF₃ group, an NO₂ group, a nitrile group, a -W(CH₂)ₙ-CF₃, -W(CF₂)ₙ-CF₃ and (CF₂)ₙ-CF₃ group in which W has the meaning already indicated and n represents an integer equal to 1, 2 or 3, a
-(CH₂)ₙ-CX₃ group in which n has the meaning already indicated and
X represents a halogen atom, or R₃ and R₄ form together an -O-CH₂-O- group and R₂, R₅ and R₆ have the meaning indicated above,
- R₇ and R₈, identical or different, represent a hydrogen atom or an alkyl radical containing 1 to 6 carbon atoms,
- R₉ represents a cycloalkyl radical containing 3 to 6 carbon atoms,
their tautomer forms, as well as their addition salts with mineral or organic bases.

2. New cycloalkyl-propen-2-amide derivatives as defined by formula (I) of claim 1, characterized in that in said formula (I),
- R₁ represents a hydrogen atom or a methyl radical,
- W represents an oxygen atom,
- R₂, R₃, R₄, R₅ and R₆, identical or different, represent a hydrogen atom, a fluorine, chlorine, bromine or iodine atom, a methyl radical, a methoxy radical, an -OCF₃ radical, a CF₃ radical, an NO₂ group, a nitrile group, a -W(CH₂)ₙ-CF₃ group in which W and n have the meaning already indicated,
or, R₃ and R₄ form together an -O-CH₂-O- group,
and R₂, R₅ and R₆ represent a hydrogen atom,
- R₇ and R₈ represent a hydrogen atom,
- R₉ represents a cyclopropyl radical,
- Y and Z have the meaning already indicated,
their tautomer forms, as well as their addition salts with mineral or organic bases.

3. New 3-cycloalkyl-propen-2-amide derivatives corresponding to formula (I) according to claim 1 or 2, characterized in that in said formula (I), R₁ represents a hydrogen atom, W represents an oxygen atom, R₂, R₃, R₄, R₅ and R₆, identical or different, represent a hydrogen atom, a fluorine, chlorine, iodine atom or an NO₂ group, or, R₃ and R₄ form together an -O-CH₂-O- group, and R₂, R₅ and R₆ represent a hydrogen atom, R₇ and R₈ represent a hydrogen atom and R₉ represents a cyclopropyl radical and Y and Z have the meaning already indicated, their tautomer forms, as well as their addition salts with mineral or organic bases.

4. N-(4-(4'-chlorophenoxy)phenyl]-2-cyano-3-cyclopropyl-3-hydroxy prop-2-enamide, as well as its addition salts with mineral or organic bases.

5. 2-cyano-3-cyclopropyl-3-hydroxy-N-[4-(4'-nitrophenoxy) phenyl] prop-2-enamide, as well as its addition salts with mineral or organic bases.

6. 2-cyano-3-cyclopropyl-3-hydroxy-N-[4-(3',4'-methylene-dioxyphenoxy)phenyl] prop-2-enamide, as well as its addition salts with mineral or organic bases.

7. 2-cyano-3-cyclopropyl-N-[4-(4'-fluorophenoxy)phenyl] 3-hydroxy prop-2-enamide, as well as its addition salts with mineral or organic bases.

8. 2-cyano-3-cyclopropyl-3-hydroxy-N-[4-(4'-iodophenoxy)-phenyl] prop-2-enamide, as well as its addition salts with mineral or organic bases.

9. Preparation process for new 3-cycloalkyl-propen-2-amide derivatives as defined by formula (I) of claim 1, as well as for their addition salts with mineral or organic bases, characterized in that a product of formula (II): 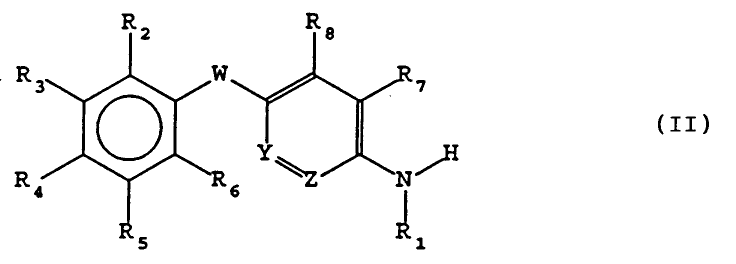 in which R₁, R₂, R₃, R₄, R₅, R₆, R₇, R₈, W, Y and Z have the meaning already indicated, is reacted with a product of formula (III):
HO-CO-CH₂-CN (III)
in order to obtain a product of formula (IV): 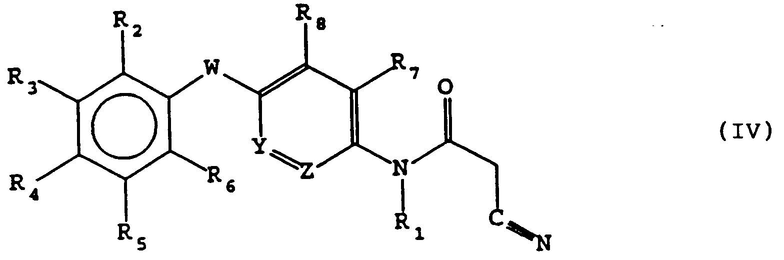 in which R₁, R₂, R₃, R₄, R₅, R₆, R₇, R₈, W, Y and Z have the meaning already indicated, then, the latter is reacted successively with sodium hydride, then with a product of formula (V):
Hal-CO-R₉ (V)
in which Hal represents a halogen atom and R₉ has the meaning already indicated, in order to obtain the sought product of formula (I), which can be salified if desired.

10. Process according to claim 9, characterized in that:
- the reaction of the product of formula (II) with the product of formula (III) is carried out in the presence of phosphorus pentachloride in an anhydrous organic solvent, such as methylene chloride or tetrahydrofuran,
- the reaction of the product of formula (IV) with sodium hydride is carried out in an anhydrous organic solvent such as tetrahydrofuran, if appropriate in the presence of a catalyst such as an imidazole.

11. Medicaments characterized in that they are constituted by the new 3-cycloalkyl-propen-2-amide derivatives, as defined by formula (I) of claim 1, as well as by their addition salts with pharmaceutically acceptable mineral or organic bases.

12. Medicaments characterized in that they are constituted by the new 3-cycloalkyl-propen-2-amide derivatives, as defined in any one of claims 2 to 8, as well as by their addition salts with pharmaceutically acceptable mineral or organic bases.

13. Pharmaceutical compositions, characterized in that they contain as active ingredient at least one of the medicaments as defined in any one of claims 11 and 12.

14. As new industrial products, the products of formula (IV): 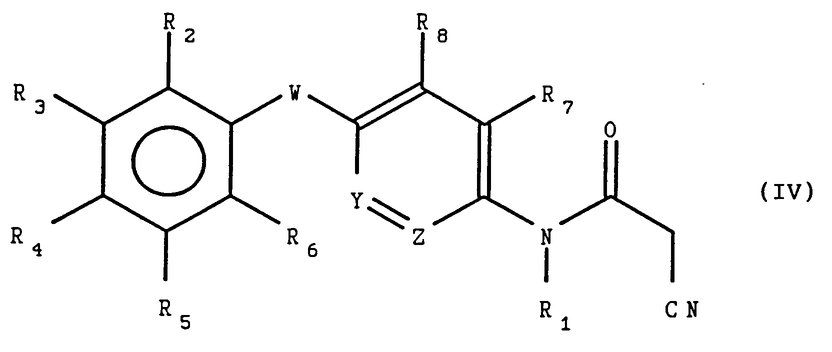 in which
R₁, R₂, R₃, R₄, R₅, R₆, R₇, R₈ and W have the meaning already indicated and one of Y and Z represents a -CH- group while the other represents a nitrogen atom.

## Claims (Claims for the following Contracting State(s): ES)

1. Process for preparing new 3-cycloalkyl-propen-2-amide derivatives corresponding to the general formula (I): 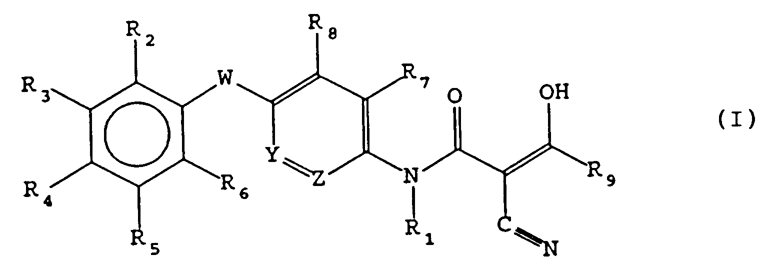 in which:
- R₁ represents a hydrogen atom or an alkyl radical containing 1 to 3 carbon atoms,
- W represents an oxygen or sulphur atom optionally oxidized in the form of the sulphoxide or sulphone,
- Y and Z represent a -CH- group or one of Y and Z represents a -CH- group while the other represents a nitrogen atom,
- R₂, R₃, R₄, R₅ and R₆, identical or different, represent a hydrogen atom, a halogen atom, an alkyl radical containing 1 to 4 carbon atoms, a methoxy radical, a methylthio radical, a -WCF₃ radical in which W has the meaning indicated above, a -CF₃ group, an NO₂ group, a nitrile group, a -W(CH₂)ₙ-CF₃,
-W(CF₂)ₙ-CF₃ and (CF₂)ₙ-CF₃ group in which W has the meaning already indicated and n represents an integer equal to 1, 2 or 3, a -(CH₂)ₙ-CX₃ group in which n has the meaning already indicated and X represents a halogen atom, or R₃ and R₄ form together an -O-CH₂-O- group and R₂, R₅ and R₆ have the meaning indicated above,
- R₇ and R₈, identical or different, represent a hydrogen atom or an alkyl radical containing 1 to 6 carbon atoms,
- R₉ represents a cycloalkyl radical containing 3 to 6 carbon atoms,
their tautomer forms, as well as their addition salts with mineral or organic bases, characterized in that a product of formula (II): 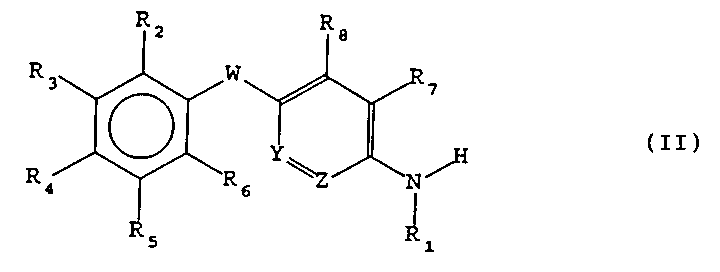 in which R₁, R₂, R₃, R₄, R₅, R₆, R₇, R₈, W, Y and Z have the meaning already indicated, is reacted with a product of formula (III):
HO-CO-CH₂-CN (III)
in order to obtain a product of formula (IV): 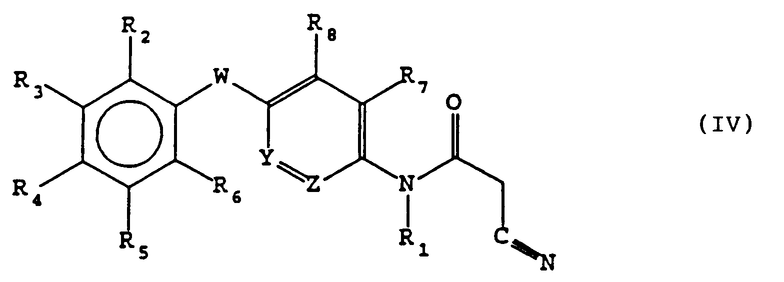 in which R₁, R₂, R₃, R₄, R₅, R₆, R₇, R₈, W, Y and Z have the meaning already indicated, then, the latter is reacted successively with sodium hydride, then with a product of formula (V):
Hal-CO-R₉ (V)
in which Hal represents a halogen atom and R₉ has the meaning already indicated, in order to obtain the sought product of formula (I), which can be salified if desired.

2. Process according to claim 1, characterized in that:
- the reaction of the product of formula (II) with the product of formula (III) is carried out in the presence of phosphorus pentachloride in an anhydrous organic solvent, such as methylene chloride or tetrahydrofuran,
- the reaction of the product of formula (IV) with sodium hydride is carried out in an anhydrous organic solvent such as tetrahydrofuran, if appropriate in the presence of a catalyst such as an imidazole.

3. Process according to claim 1 or 2, characterized in that a product of formula (II) is used at the start in which:
- R₁ represents a hydrogen atom or a methyl radical,
- W represents an oxygen atom,
- R₂, R₃, R₄, R₅ and R₆, identical or different, represent a hydrogen atom, a fluorine, chlorine, bromine or iodine atom, a methyl radical, a methoxy radical, an -OCF₃ radical, a CF₃ radical, an NO₂ group, a nitrile group, a -W(CH₂)ₙ-CF₃ group in which W and n have the meaning already indicated,
or, R₃ and R₄ form together an -O-CH₂-O- group,
and R₂, R₅ and R₆ represent a hydrogen atom,
- R₇ and R₈ represent a hydrogen atom,
- R₉ represents a cyclopropyl radical,
- Y and Z have the meaning already indicated,
and a product of formula (V) in which R₉ represents a cyclopropyl radical.

4. Process according to any one of claims 1 to 3, characterized in that a product of formula (II) is used at the start in which:
R₁ represents a hydrogen atom, W represents an oxygen atom, R₂, R₃, R₄, R₅ and R₆, identical or different, represent a hydrogen atom, a fluorine, chlorine, iodine atom or an NO₂ group, or, R₃ and R₄ form together an -O-CH₂-O- group, and R₂, R₅ and R₆ represent a hydrogen atom, R₇ and R₈ represent a hydrogen atom and Y and Z have the meaning already indicated,
and a product of formula (V) in which R₉ represents a cyclopropyl radical.

## Claims (Claims for the following Contracting State(s): GR)

1. Process for preparing new 3-cycloalkyl-propen-2-amide derivatives corresponding to the general formula (I): 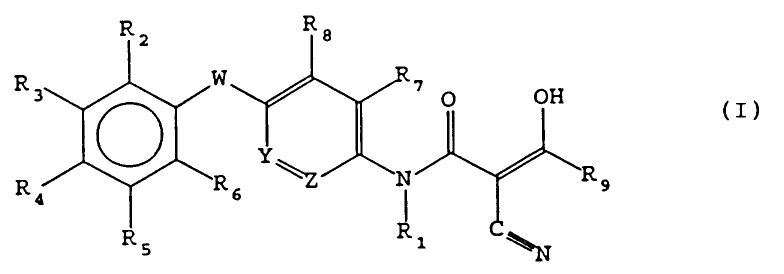 in which:
- R₁ represents a hydrogen atom or an alkyl radical containing 1 to 3 carbon atoms,
- W represents an oxygen or sulphur atom optionally oxidized in the form of the sulphoxide or sulphone,
- Y and Z represent a -CH- group or one of Y and Z represents a -CH- group while the other represents a nitrogen atom,
- R₂, R₃, R₄, R₅ and R₆, identical or different, represent a hydrogen atom, a halogen atom, an alkyl radical containing 1 to 4 carbon atoms, a methoxy radical, a methylthio radical, a -WCF₃ radical in which W has the meaning indicated above, a -CF₃ group, an NO₂ group, a nitrile group, a -W(CH₂)ₙ-CF₃,
-W(CF₂)ₙ-CF₃ and (CF₂)ₙ-CF₃ group in which W has the meaning already indicated and n represents an integer equal to 1, 2 or 3, a -(CH₂)ₙ-CX₃ group in which n has the meaning already indicated and X represents a halogen atom, or R₃ and R₄ form together an -O-CH₂-O- group and R₂, R₅ and R₆ have the meaning indicated above,
- R₇ and R₈, identical or different, represent a hydrogen atom or an alkyl radical containing 1 to 6 carbon atoms,
- R₉ represents a cycloalkyl radical containing 3 to 6 carbon atoms,
their tautomer forms, as well as their addition salts with mineral or organic bases, characterized in that a product of formula (II): 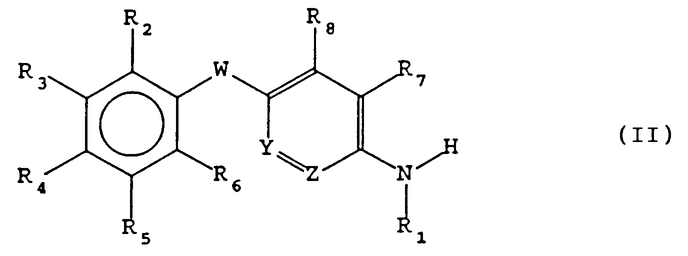 in which R₁, R₂, R₃, R₄, R₅, R₆, R₇, R₈, W, Y and Z have the meaning already indicated, is reacted with a product of formula (III):
HO-CO-CH₂-CN (III)
in order to obtain a product of formula (IV): 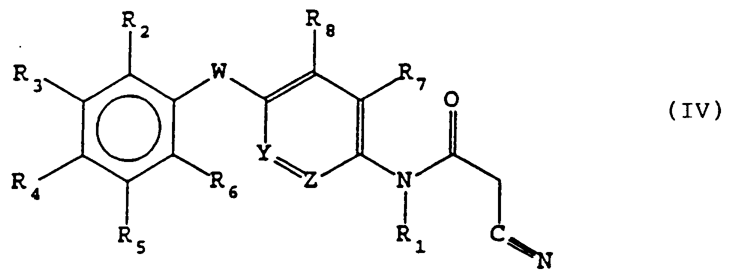 in which R₁, R₂, R₃, R₄, R₅, R₆, R₇, R₈, W, Y and Z have the meaning already indicated, then, the latter is reacted successively with sodium hydride, then with a product of formula (V):
Hal-CO-R₉ (V)
in which Hal represents a halogen atom and R₉ has the meaning already indicated, in order to obtain the sought product of formula (I), which can be salified if desired.

2. Process according to claim 1, characterized in that:
- the reaction of the product of formula (II) with the product of formula (III) is carried out in the presence of phosphorus pentachloride in an anhydrous organic solvent, such as methylene chloride or tetrahydrofuran,
- the reaction of the product of formula (IV) with sodium hydride is carried out in an anhydrous organic solvent such as tetrahydrofuran, if appropriate in the presence of a catalyst such as an imidazole.

3. Process according to claim 1 or 2, characterized in that a product of formula (II) is used at the start in which:
- R₁ represents a hydrogen atom or a methyl radical,
- W represents an oxygen atom,
- R₂, R₃, R₄, R₅ and R₆, identical or different, represent a hydrogen atom, a fluorine, chlorine, bromine or iodine atom, a methyl radical, a methoxy radical, an -OCF₃ radical, a CF₃ radical, an NO₂ group, a nitrile group, a -W(CH₂)ₙ-CF₃ group in which W and n have the meaning already indicated,
or, R₃ and R₄ form together an -O-CH₂-O- group,
and R₂, R₅ and R₆ represent a hydrogen atom,
- R₇ and R₈ represent a hydrogen atom,
- Y and Z have the meaning already indicated,
and a product of formula (V) in which R₉ represents a cyclopropyl radical.

4. Process according to any one of claims 1 to 3, characterized in that a product of formula (II) is used at the start in which:
R₁ represents a hydrogen atom, W represents an oxygen atom, R₂, R₃, R₄, R₅ and R₆, identical or different, represent a hydrogen atom, a fluorine, chlorine, iodine atom or an NO₂ group, or, R₃ and R₄ form together an -O-CH₂-O- group, and R₂, R₅ and R₆ represent a hydrogen atom, R₇ and R₈ represent a hydrogen atom and Y and Z have the meaning already indicated,
and a product of formula (V) in which R₉ represents a cyclopropyl radical.

5. Process according to any one of claims 1 to 4 characterized in that at the start a product of formula (II) and a product of formula (V) are used, chosen in such a way that any one of the products of which the names follow is prepared:
- N-[4-(4'-chlorophenoxy)phenyl]-2-cyano-3-cyclopropyl-3-hydroxy prop-2-enamide, as well as its addition salts with mineral or organic bases,
- 2-cyano-3-cyclopropyl-3-hydroxy-N-[4-(4'-nitrophenoxy)-phenyl] prop-2-enamide, as well as its addition salts with mineral or organic bases,
- 2-cyano-3-cyclopropyl-3-hydroxy-N-[4-(3',4'-methylene-dioxyphenoxy)phenyl] prop-2-enamide, as well as its addition salts with mineral or organic bases,
- 2-cyano-3-cyclopropyl-N-[4-(4'-fluorophenoxy)phenyl] 3-hydroxy prop-2-enamide, as well as its addition salts with mineral or organic bases,
- 2-cyano-3-cyclopropyl-3-hydroxy-N-[4-(4,-iodophenoxy)-phenyl] prop-2-enamide, as well as its addition salts with mineral or organic bases.

6. Preparation process for pharmaceutical compositions, characterized in that at least one of the products of formula (I) as defined in claim 1 or at least one of their pharmaceutically acceptable salts is used as active ingredient in a form intended for this use.

7. Preparation process for pharmaceutical compositions, characterized in that at least one of the products of formula (I) as defined in one of claims 2 to 5 or at least one of their pharmaceutically acceptable salts is used as active ingredient in a form intended for this use.

8. As new industrial products, the products of formula (IV): 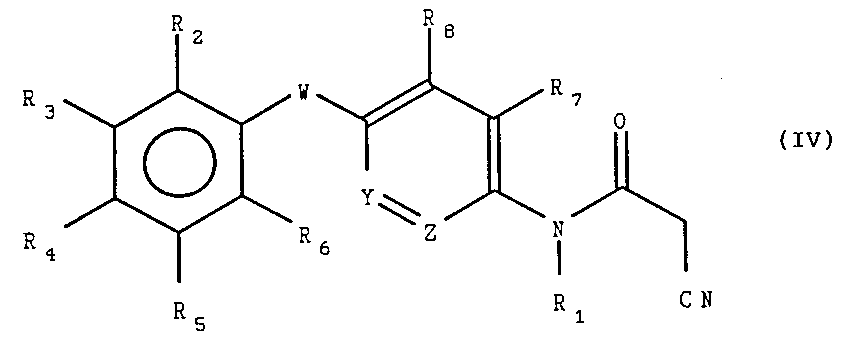 in which
R₁, R₂, R₃, R₄, R₅, R₆, R₇, R₈ and W have the meaning already indicated and one of Y and Z represents a -CH- group while the other represents a nitrogen atom.

## Patentansprüche (Patentansprüche für folgende(n) Vertragsstaat(en): AT, BE, CH, DE, DK, FR, GB, IE, IT, LI, LU, NL, PT, SE)

1. Neue 3-Cycloalkylpropen-2-amidderivate der allgemeinen Formel (I) 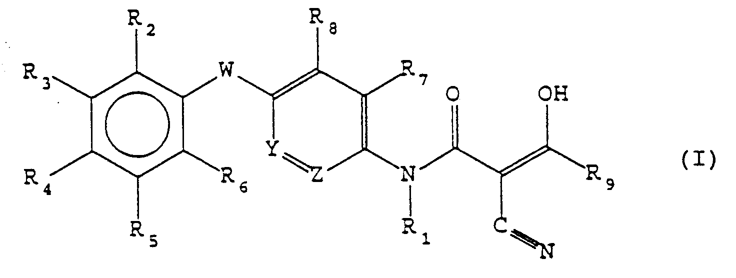 worin:
- R₁ ein Wasserstoffatom oder einen Alkylrest mit 1 bis 3 Kohlenstoffatomen bedeutet,
- W ein Sauerstoff- oder Schwefelatom, gegebenenfalls in Form eines Sulfoxids oder Sulfons oxidiert, bedeutet,
- Y und Z eine Gruppierung -CH- bedeuten oder ein Rest von Y und Z eine Gruppierung -CH- bedeutet, während der andere ein Stickstoffatom bedeutet,
- R₂, R₃, R₄, R₅ und R₆, die gleich oder verschieden sein können, ein Wasserstoffatom, ein Halogenatom, einen Alkylrest mit 1 bis 4 Kohlenstoffatomen, eine Methoxygruppe, eine Methylthiogruppe, einen -WCF₃-Rest, worin W wie vorstehend definiert ist, eine -CF₃-Gruppe, eine NO₂-Gruppe, eine Nitrilgruppe, eine Gruppierung -W(CH₂)ₙ-CF₃, -W(CF₂)ₙ-CF₃ und (CF₂)ₙ-CF₃, worin W wie vorstehend definiert ist, und n eine ganze Zahl von 1, 2 oder 3 bedeutet, eine Gruppierung -(CH₂)ₙ-CX₃, worin n wie vorstehend definiert ist, und X ein Halogenatom bedeutet, bedeuten, oder R₃ und R₄ zusammen eine Gruppierung -O-CH₂-O- bilden, und R₂, R₅ und R₆ wie vorstehend definiert sind,
- R₇ und R₈, die gleich oder verschieden sein können, ein Wasserstoffatom oder einen Alkylrest mit 1 bis 6 Kohlenstoffatomen bedeuten,
- R₉ einen Cycloalkylrest mit 3 bis 6 Kohlenstoffatomen bedeutet,
ihre tautomeren Formen sowie ihre Additionssalze mit mineralischen oder organischen Basen.

2. Neue Cycloalkylpropen-2-amidderivate, wie durch die Formel (I) in Anspruch 1 definiert, dadurch **gekennzeichnet,** daß in der Formel (I)
- R₁ ein Wasserstoffatom oder eine Methylgruppe bedeutet,
- W ein Sauerstoffatom bedeutet,
- R₂, R₃, R₄, R₅ und R₆, die gleich oder verschieden sein können, ein Wasserstoffatom, ein Fluor-, Chlor-, Brom- oder Iodatom, eine Methylgruppe, eine Methoxygruppe, eine -OCF₃-Gruppe, eine -CF₃-Gruppe, eine NO₂-Gruppe, eine Nitrilgruppe, eine Gruppierung -W(CH₂)ₙ-CF₃, worin W und n wie vorstehend definiert sind, bedeuten, oder R₃ und R₄ zusammen eine Gruppierung -O-CH₂-O- bilden, und R₂, R₅ und R₆ ein Wasserstoffatom bedeuten,
- R₇ und R₈ ein Wasserstoffatom bedeuten,
- R₉ einen Cyclopropylrest bedeutet,
- Y und Z wie vorstehend definiert sind,
ihre tautomeren Formen sowie ihre Additionssalze mit mineralischen oder organischen Basen.

3. Neue 3-Cycloalkylpropen-2-amidderivate der Formel (I) nach Anspruch 1 oder 2, dadurch **gekennzeichnet,** daß in der Formel (I) R₁ ein Wasserstoffatom bedeutet, W ein Sauerstoffatom bedeutet, R₂, R₃, R₄, R₅ und R₆, die gleich oder verschieden sein können, ein Wasserstoffatom, ein Fluor-, Chlor-, Iodatom oder eine NO₂-Gruppe bedeuten, oder R₃ und R₄ zusammen eine Gruppierung -O-CH₂-O- bilden, und R₂, R₅ und R₆ ein Wasserstoffatom bedeuten, R₇ und R₈ ein Wasserstoffatom bedeuten, und R₉ einen Cyclopropylrest bedeutet, und Y und Z wie vorstehend definiert sind, ihre tautomeren Formen sowie ihre Additionssalze mit mineralischen oder organischen Basen.

4. N-[4-(4'-Chlorphenoxy)phenyl]-2-cyano-3-cyclopropyl-3-hydroxyprop-2-enamid sowie die Additionssalze davon mit mineralischen oder organischen Basen.

5. 2-Cyano-3-cyclopropyl-3-hydroxy-N-[4-(4'-nitrophenoxy)phenyl]prop-2-enamid sowie die Additionssalze davon mit mineralischen oder organischen Basen.

6. 2-Cyano-3-cyclopropyl-3-hydroxy-N-[4-(3',4'-methylendioxyphenoxy)phenyl]prop-2-enamid sowie die Additionssalze davon mit mineralischen oder organischen Basen.

7. 2-Cyano-3-cyclopropyl- N-[4-(4'-fluorphenoxy)phenyl]-3-hydroxyprop-2-enamid sowie die Additionssalze davon mit mineralischen oder organischen Basen.

8. 2-Cyano-3-cyclopropyl-3-hydroxy-N-[4-(4'-iodphenoxy)phenyl]prop-2-enamid sowie die Additionssalze davon mit mineralischen oder organischen Basen.

9. Verfahren zur Herstellung von neuen 3-Cycloalkylpropen-2-amidderivaten, wie durch Formel (I) in Anspruch 1 definiert, sowie ihrer Additionssalze mit mineralischen oder organischen Basen, dadurch **gekennzeichnet,** daß man eine Verbindung der Formel (II) 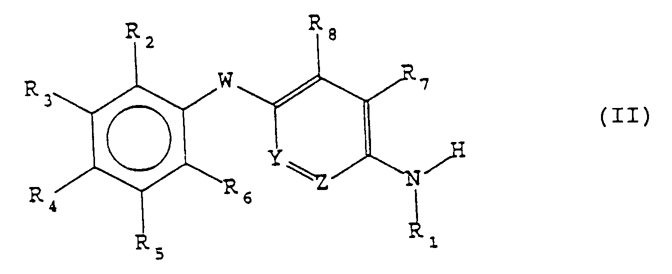 worin R₁, R₂, R₃, R₄, R₅, R₆, R₇, R₈, W, Y und Z wie vorstehend definiert sind, mit einer Verbindung der Formel (III)
HO-CO-CH₂-CN (III)
umsetzt, um eine Verbindung der Formel (IV) 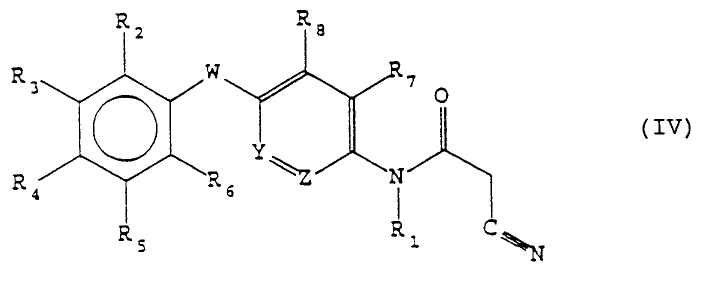 zu erhalten, worin R₁, R₂, R₃, R₄, R₅, R₆, R₇, R₈, W, Y und Z wie vorstehend definiert sind, anschließend diese zuletzt genannte Verbindung sukzessive mit Natriumhydrid, dann mit einer Verbindung der Formel (V)
Hal-CO-R₉ (V)
worin Hal ein Halogenatom bedeutet, und R₉ wie vorstehend definiert ist, umsetzt, um die gewünschte Verbindung der Formel (I) zu erhalten, die gegebenenfalls in ein Salz überführt werden kann.

10. Verfahren nach Anspruch 9, dadurch **gekennzeichnet,** daß
- die Umsetzung der Verbindung der Formel (II) mit der Verbindung der Formel (III) in Gegenwart von Phosphorpentachlorid in einem wasserfreien organischen Lösungsmittel, wie Methylenchlorid oder Tetrahydrofuran, durchgeführt wird,
- die Umsetzung der Verbindung der Formel (IV) mit Natriumhydrid in einem wasserfreien organischen Lösungsmittel, wie Tetrahydrofuran, gegebenenfalls in Gegenwart eines Katalysators, wie eines Imidazols, durchgeführt wird.

11. Medikamente, dadurch **gekennzeichnet,** daß sie aus den neuen 3-Cycloalkylpropen-2-amidderivaten, wie durch Formel (I) in Anspruch 1 definiert, sowie ihren Additionssalzen mit mineralischen oder organischen pharmazeutischen verträglichen Basen bestehen.

12. Medikamente, dadurch **gekennzeichnet,** daß sie aus den neuen 3-Cycloalkylpropen-2-amidderivaten, wie in einem der Ansprüche 2 bis 8 definiert, sowie ihren Additionssalzen mit mineralischen oder organischen pharmazeutisch verträglichen Basen bestehen.

13. Pharmazeutische Zusammensetzungen, dadurch **gekennzeichnet,** daß sie als Wirkstoff mindestens eines der Medikamente, wie in einem der Ansprüche 11 und 12 definiert, umfassen.

14. Verbindungen der Formel (IV) 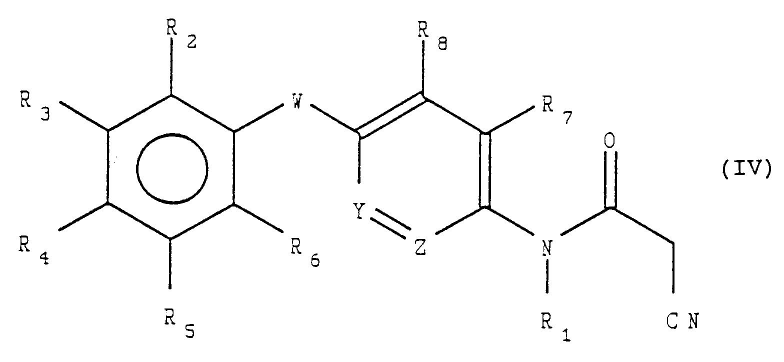 worin R₁, R₂, R₃, R₄, R₅, R₆, R₇, R₈ und W wie vorstehend definiert sind, und einer der Reste von Y und Z eine Gruppierung -CH- bedeutet, während der andere ein Stickstoffatom bedeutet, als neue industrielle Produkte.

## Patentansprüche (Patentansprüche für folgende(n) Vertragsstaat(en): ES)

1. Verfahren zur Herstellung von neuen 3-Cycloalkylpro-pen-2-amidderivaten der allgemeinen Formel (I) 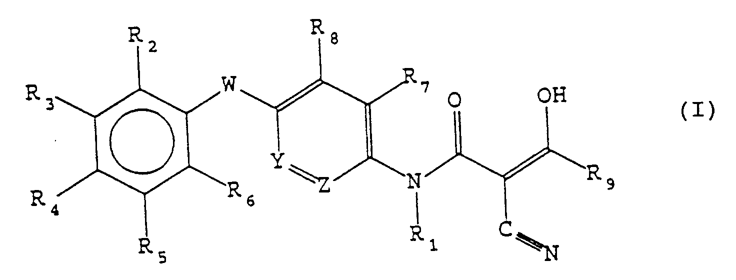 worin
- R₁ ein Wasserstoffatom oder einen Alkylrest mit 1 bis 3 Kohlenstoffatomen bedeutet,
- W ein Sauerstoff- oder Schwefelatom, gegebenenfalls in Form eines Sulfoxids oder Sulfons oxidiert, bedeutet,
- Y und Z eine Gruppierung -CH- bedeuten oder ein Rest von Y und Z eine Gruppierung -CH- bedeutet, während der andere ein Stickstoffatom bedeutet,
- R₂, R₃, R₄, R₅ und R₆, die gleich oder verschieden sein können, ein Wasserstoffatom, ein Halogenatom, einen Alkylrest mit 1 bis 4 Kohlenstoffatomen, eine Methoxygruppe, eine Methylthiogruppe, einen -WCF₃-Rest, worin W wie vorstehend definiert ist, eine -CF₃-Gruppe, eine NO₂-Gruppe, eine Nitrilgruppe, eine Gruppierung -W(CH₂)ₙ-CF₃, -W(CF₂)ₙ-CF₃ und (CF₂)ₙ-CF₃, worin W wie vorstehend definiert ist, und n eine ganze Zahl von 1, 2 oder 3 bedeutet, eine Gruppierung -(CH₂)ₙ-CX₃, worin n wie vorstehend definiert ist, und X ein Halogenatom bedeutet, bedeuten, oder R₃ und R₄ zusammen eine Gruppierung -O-CH₂-O- bilden, und R₂, R₅ und R₆ wie vorstehend definiert sind,
- R₇ und R₈, die gleich oder verschieden sein können, ein Wasserstoffatom oder einen Alkylrest mit 1 bis 6 Kohlenstoffatomen bedeuten,
- R₉ einen Cycloalkylrest mit 3 bis 6 Kohlenstoffatomen bedeutet,
ihrer tautomeren Formen sowie ihrer Additionssalze mit mineralischen oder organischen Basen, dadurch **gekennzeichnet,** daß man eine Verbindung der Formel (II) 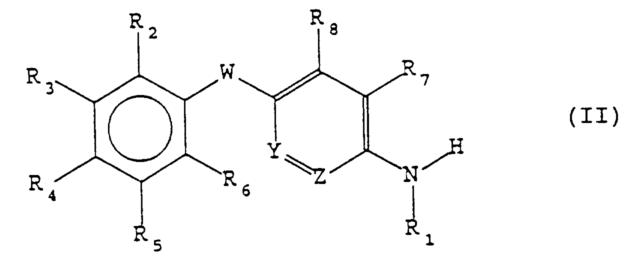 worin R₁, R₂, R₃, R₄, R₅, R₆, R₇, R₈, W, Y und Z wie vorstehend definiert sind, mit einer Verbindung der Formel (III)
HO-CO-CH₂-CN (III)
umsetzt, um eine Verbindung der Formel (IV) 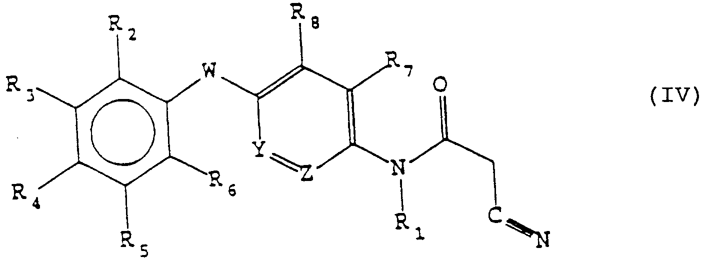 zu erhalten, worin R₁, R₂, R₃, R₄, R₅, R₆, R₇, R₈, W, Y und Z wie vorstehend definiert sind, anschließend diese zuletzt genannte Verbindung sukzessive mit Natriumhydrid, dann mit einer Verbindung der Formel (V)
Hal-CO-R₉ (V)
worin Hal ein Halogenatom bedeutet, und R₉ wie vorstehend definiert ist, umsetzt, um die gewünschte Verbindung der Formel (I) zu erhalten, die gegebenenfalls in ein Salz überführt werden kann.

2. Verfahren nach Anspruch 1, dadurch **gekennzeichnet,** daß
- die Umsetzung der Verbindung der Formel (II) mit der Verbindung der Formel (III) in Gegenwart von Phosphorpentachlorid in einem wasserfreien organischen Lösungsmittel, wie Methylenchlorid oder Tetrahydrofuran, durchgeführt wird,
- die Umsetzung der Verbindung der Formel (IV) mit Natriumhydrid in einem wasserfreien organischen Lösungsmittel, wie Tetrahydrofuran, gegebenenfalls in Gegenwart eines Katalysators, wie eines Imidazols, durchgeführt wird.

3. Verfahren nach Anspruch 1 oder 2, dadurch **gekennzeichnet,** daß man als Ausgangsverbindung eine Verbindung der Formel (II) worin:
- R₁ ein Wasserstoffatom oder einen Methylrest bedeutet,
- W ein Sauerstoffatom bedeutet,
- R₂, R₃, R₄, R₅ und R₆, die gleich oder verschieden sein können, ein Wasserstoffatom, ein Fluor-, Chlor-, Brom- oder Iodatom, eine Methylgruppe, eine Methoxygruppe, eine -OCF₃-Gruppe, eine -CF₃-Gruppe, eine NO₂-Gruppe, eine Nitrilgruppe, eine Gruppierung -W(CH₂)ₙ-CF₃, worin W und n wie vorstehend definiert sind, bedeuten, oder R₃ und R₄ zusammen eine Gruppierung -O-CH₂-O- bilden, und R₂, R₅ und R₆ ein Wasserstoffatom bedeuten,
- R₇ und R₈ ein Wasserstoffatom bedeuten,
- Y und Z wie vorstehend definiert sind,
und eine Verbindung der Formel (V), worin R₉ eine Cyclopropylgruppe bedeutet, verwendet.

4. Verfahren nach einem der Ansprüche 1 bis 3, dadurch **gekennzeichnet,** daß man als Ausgangsverbindung eine Verbindung der Formel (II), worin R₁ ein Wasserstoffatom bedeutet, W ein Sauerstoffatom bedeutet, R₂, R₃, R₄, R₅ und R₆, die gleich oder verschieden sein können, ein Wasserstoffatom, ein Fluor-, Chlor-, Iodatom oder eine NO₂-Gruppe bedeuten, oder R₃ und R₄ zusammen eine Gruppierung -O-CH₂-O- bilden, und R₂, R₅ und R₆ ein Wasserstoffatom bedeuten, R₇ und R₈ ein Wasserstoffatom bedeuten und Y und Z wie vorstehend definiert sind, und eine Verbindung der Formel (V), worin R₉ eine Cyclopropylgruppe bedeutet, verwendet.

## Patentansprüche (Patentansprüche für folgende(n) Vertragsstaat(en): GR)

1. Verfahren zur Herstellung von neuen 3-Cycloalkylpropen-2-amidderivaten der allgemeinen Formel (I) 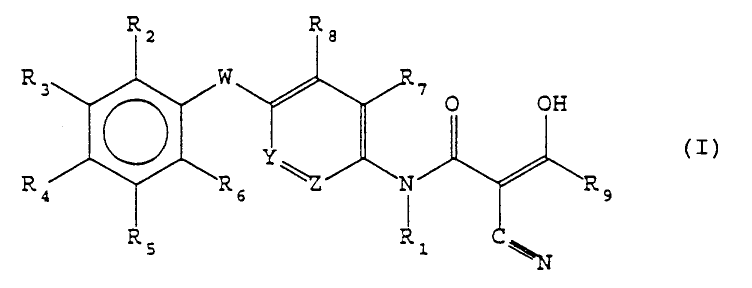 worin
- R₁ ein Wasserstoffatom oder einen Alkylrest mit 1 bis 3 Kohlenstoffatomen bedeutet,
- W ein Sauerstoff- oder Schwefelatom, gegebenenfalls in Form eines Sulfoxids oder Sulfons oxidiert, bedeutet,
- Y und Z eine Gruppierung -CH- bedeuten oder ein Rest von Y und Z eine Gruppierung -CH- bedeutet, während der andere ein Stickstoffatom bedeutet,
- R₂, R₃, R₄, R₅ und R₆, die gleich oder verschieden sein können, ein Wasserstoffatom, ein Halogenatom, einen Alkylrest mit 1 bis 4 Kohlenstoffatomen, eine Methoxygruppe, eine Methylthiogruppe, einen -WCF₃-Rest, worin W wie vorstehend definiert ist, eine -CF₃-Gruppe, eine NO₂-Gruppe, eine Nitrilgruppe, eine Gruppierung -W(CH₂)ₙ-CF₃, -W(CF₂)ₙ-CF₃ und (CF₂)ₙ-CF₃, worin W wie vorstehend definiert ist, und n eine ganze Zahl von 1, 2 oder 3 bedeutet, eine Gruppierung -(CH₂)ₙ-CX₃, worin n wie vorstehend definiert ist, und X ein Halogenatom bedeutet, bedeuten, oder R₃ und R₄ zusammen eine Gruppierung -O-CH₂-O- bilden, und R₂, R₅ und R₆ wie vorstehend definiert sind,
- R₇ und R₈, die gleich oder verschieden sein können, ein Wasserstoffatom oder einen Alkylrest mit 1 bis 6 Kohlenstoffatomen bedeuten,
- R₉ einen Cycloalkylrest mit 3 bis 6 Kohlenstoffatomen bedeutet,
ihrer tautomeren Formen sowie ihrer Additionssalze mit mineralischen oder organischen Basen, dadurch **gekennzeichnet,** daß man eine Verbindung der Formel (II) 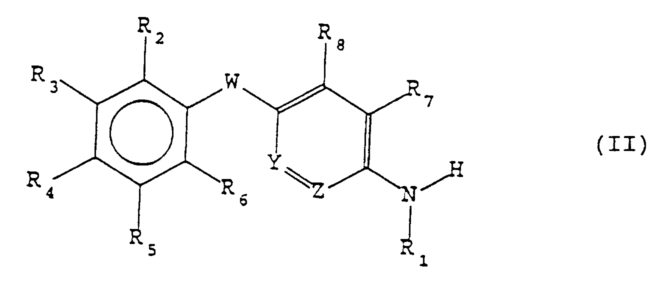 worin R₁, R₂, R₃, R₄, R₅, R₆, R₇, R₈, W, Y und Z wie vorstehend definiert sind, mit einer Verbindung der Formel (III)
HO-CO-CH₂-CN (III)
umsetzt, um eine Verbindung der Formel (IV) 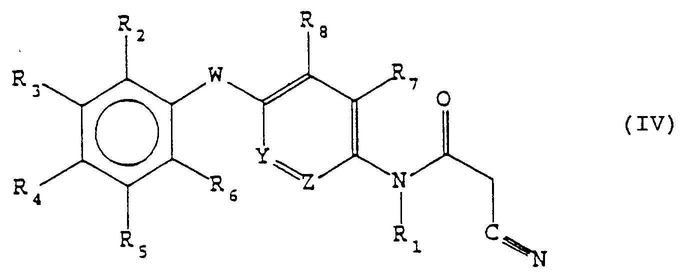 zu erhalten, worin R₁, R₂, R₃, R₄, R₅, R₆, R₇, R₈, W, Y und Z wie vorstehend definiert sind, anschließend diese zuletzt genannte Verbindung sukzessive mit Natriumhydrid, dann mit einer Verbindung der Formel (V)
Hal-CO-R₉ (V)
worin Hal ein Halogenatom bedeutet, und R₉ wie vorstehend definiert ist, umsetzt, um die gewünschte Verbindung der Formel (I) zu erhalten, die gegebenenfalls in ein Salz überführt werden kann.

2. Verfahren nach Anspruch 1, dadurch **gekennzeichnet,** daß
- die Umsetzung der Verbindung der Formel (II) mit der Verbindung der Formel (III) in Gegenwart von Phosphorpentachlorid in einem wasserfreien organischen Lösungsmittel, wie Methylenchlorid oder Tetrahydrofuran, durchgeführt wird,
- die Umsetzung der Verbindung der Formel (IV) mit Natriumhydrid in einem wasserfreien organischen Lösungsmittel, wie Tetrahydrofuran, gegebenenfalls in Gegenwart eines Katalysators, wie eines Imidazols, durchgeführt wird.

3. Verfahren nach Anspruch 1 oder 2, dadurch **gekennzeichnet,** daß man als Ausgangsverbindung eine Verbindung der Formel (II) worin:
- R₁ ein Wasserstoffatom oder einen Methylrest bedeutet,
- W ein Sauerstoffatom bedeutet,
- R₂, R₃, R₄, R₅ und R₆, die gleich oder verschieden sein können, ein Wasserstoffatom, ein Fluor-, Chlor-, Brom- oder Iodatom, eine Methylgruppe, eine Methoxygruppe, eine -OCF₃-Gruppe, eine -CF₃-Gruppe, eine NO₂-Gruppe, eine Nitrilgruppe, eine Gruppierung -W(CH₂)ₙ-CF₃, worin W und n wie vorstehend definiert sind, bedeuten, oder R₃ und R₄ zusammen eine Gruppierung -O-CH₂-O- bilden, und R₂, R₅ und R₆ ein Wasserstoffatom bedeuten,
- R₇ und R₈ ein Wasserstoffatom bedeuten,
- Y und Z wie vorstehend definiert sind,
und eine Verbindung der Formel (V), worin R₉ eine Cyclopropylgruppe bedeutet, verwendet.

4. Verfahren nach einem der Ansprüche 1 bis 3, dadurch **gekennzeichnet,** daß man als Ausgangsverbindung eine Verbindung der Formel (II), worin R₁ ein Wasserstoffatom bedeutet, W ein Sauerstoffatom bedeutet, R₂, R₃, R₄, R₅ und R₆, die gleich oder verschieden sein können, ein Wasserstoffatom, ein Fluor-, Chlor-, Iodatom oder eine NO₂-Gruppe bedeuten, oder R₃ und R₄ zusammen eine Gruppierung -O-CH₂-O- bilden, und R₂, R₅ und R₆ ein Wasserstoffatom bedeuten, R₇ und R₈ ein Wasserstoffatom bedeuten, und Y und Z wie vorstehend definiert sind, und eine Verbindung der Formel (V), worin R₉ eine Cyclopropylgruppe bedeutet, verwendet.

5. Verfahren nach einem der Ansprüche 1 bis 4, dadurch **gekennzeichnet,** daß man als Ausgangsverbindung eine Verbindung der Formel (II) und eine Verbindung der Formel (V) so ausgewählt verwendet, daß man eine der nachstehenden Verbindungen herstellt:
- N-[4-(4'-Chlorphenoxy)phenyl]-2-cyano-3-cyclopropyl-3-hy-droxyprop-2-enamid sowie die Additionssalze davon mit mineralischen oder organischen Basen,
- 2-Cyano-3-cyclopropyl-3-hydroxy-N-[4-(4'-nitrophenoxy)-phenyl]prop-2-enamid sowie die Additionssalze davon mit mineralischen oder organischen Basen,
- 2-Cyano-3-cyclopropyl-3-hydroxy-N-[4-(3',4'-methylendioxyphenoxy)phenyl]prop-2-enamid sowie die Additionssalze davon mit mineralischen oder organischen Basen,
- 2-Cyano-3-cyclopropyl-N-[4-(4'-fluorphenoxy)phenyl]-3-hydroxyprop-2-enamid sowie die Additionssalze davon mit mineralischen oder organischen Basen,
- 2-Cyano-3-cyclopropyl-3-hydroxy-N-[4-(4'-iodphenoxy)phenyl]prop-2-enamid sowie die Additionssalze davon mit mineralischen oder organischen Basen.

6. Verfahren zur Herstellung von pharmazeutischen Zusammensetzungen, dadurch **gekennzeichnet,** daß man als Wirkstoff mindestens eine der Verbindungen der Formel (I), wie in Anspruch 1 definiert, oder mindestens eines ihrer pharmazeutisch verträglichen Salze in einer für diese Verwendung bestimmten Form verwendet.

7. Verfahren zur Herstellung von pharmazeutischen Zusammensetzungen, dadurch **gekennzeichnet,** daß man als Wirkstoff mindestens eine der Verbindungen der Formel (I), wie in einem der Ansprüche 2 bis 5 definiert, oder mindestens eines ihrer pharmazeutisch verträglichen Salze in einer für diese Verwendung bestimmten Form verwendet.

8. Verbindungen der Formel (IV) 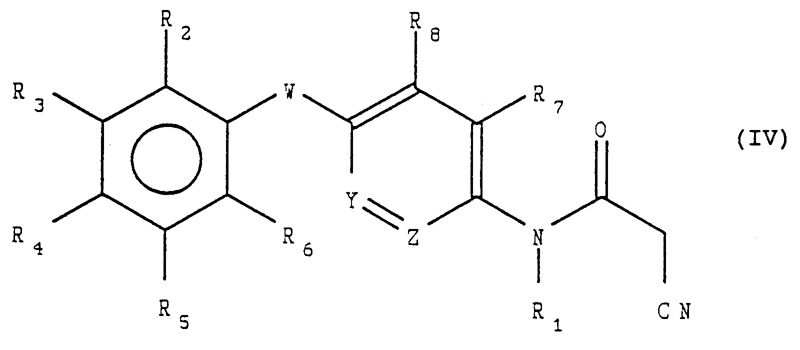 worin R₁, R₂, R₃, R₄, R₅, R₆, R₇, R₈ und W wie vorstehend definiert sind, und einer der Reste von Y und Z eine Gruppierung -CH- bedeutet, während der andere ein Stickstoffatom bedeutet, als neue industrielle Produkte.
